(19) Europäisches Patentamt
European Patent Office
Office européen des brevets

(11) Veröffentlichungsnummer: **0 075 850**
**B1**

(12) # EUROPÄISCHE PATENTSCHRIFT

(45) Veröffentlichungstag der Patentschrift: **18.12.85**

(21) Anmeldenummer: **82108751.7**

(22) Anmeldetag: **22.09.82**

(51) Int. Cl.⁴: **C 07 D 473/06**, C 07 D 473/08, A 61 K 31/52 // C07C21/04, C07C31/42, C07D303/08, C07D317/16, C07D317/20

(54) **Arzneimittel, darin enthaltene vicinale Dihydroxyalkylxanthine, Herstellungsverfahren für diese Xanthinverbindungen und dafür geeignete Zwischenprodukte.**

(30) Priorität: **26.09.81 DE 3138397**

(43) Veröffentlichungstag der Anmeldung: **06.04.83 Patentblatt 83/14**

(45) Bekanntmachung des Hinweises auf die Patenterteilung: **18.12.85 Patentblatt 85/51**

(84) Benannte Vertragsstaaten: **AT BE CH DE FR GB IT LI LU NL SE**

(56) Entgegenhaltungen:
**DE-A-2 716 402**
**FR-A-2 480 286**

**ARZNEIMITTELFORSCHUNG, Band 22, Nr. 9, 1972, AULENDORF (DE), H.J. HINZE: "Zur Pharmakokinetik von 3,7-Dimethyl-1-(5-oxo-hexyl)-xanthin am Menschen", Seiten 1492-1495**
**ARZNEIMITTELFORSCHUNG, Band 22, Nr. 7, 1972, AULENDORF (DE), H.J. HINZE et al.: "Struktur der Ausscheidungsprodukte des 3,7-Dimethyl-1-(5-oxo-hexyl)-xanthins beim Menschen", Seiten 1144-1151**
**CHEMICAL ABSTRACTS, vol. 88, Nr. 7, 13 Februar 1978, Seite 556, Zusammenfassung Nr. 50931s, COLUMBUS OHIO (US)**

(73) Patentinhaber: **HOECHST AKTIENGESELLSCHAFT, Postfach 80 03 20, D-6230 Frankfurt am Main 80 (DE)**

(72) Erfinder: **Furrer, Harald, Dr., Breslauer Strasse 27, D-6233 Kelkheim (Taunus) (DE)**
Erfinder: **Anagnostopulos, Hiristo, Kantstrasse 22, D-6204 Taunusstein (DE)**
Erfinder: **Gebert, Ulrich, Dr., Albert-Lortzing-Strasse 2, D-6233 Kelkheim (Taunus) (DE)**
Erfinder: **Hinze, Heinz-Joachim, Dr., Tannenring 84, D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Arzneimittel, die sich inbesondere zur Behandlung obstruktiver Atemwegserkrankungen eignen, die in ihnen enthaltenen pharmakologisch aktiven vicinalen Dihydroxyalkylxanthine, Verfahren zur Herstellung dieser Xanthinderivate und Zwischenprodukte hierzu.

Unter den bislang bekannten Bronchospasmolytika nehmen die als Phosphodiesterase-Inhibitoren agierenden Xanthinverbindungen eine Vorzugsstellung ein (vergl. B. Hellwig, Moderne Arzneimittel), Stuttgart 1980, S. 1274), da sie keine $\beta_2$-sympathikomimetische Aktivität besitzen und sich deshalb besonders gut für die bei chronisch-obstruktiven Atemwegsleiden stets notwendige Dauertherapie eignen. Aus dieser Stoffgruppe stellt das natürlich vorkommende Xanthinderivat Theophyllin (1,3-Dimethylxanthin) seit nunmehr einigen Jahrzehnten unangefochten das Mittel der Wahl in der Asthmatherapie dar. Seiner klinisch gut etablierten Wirkung stehen jedoch die Nachteile einer sehr engen therapeutischen Breite, gravierender Nebenwirkungen im gastrointestinalen, kardiovaskulären und renalen Bereich sowie im zentralen Nervensystem und seiner nur enteralen Anwendbarkeit aufgrund mangelnder Wasserlöslichkeit gegenüber, die den Wunsch der Kliniker und die Suche der pharmazeutischen Forschung nach Präparaten mit einer grösseren Therapiesicherheit begründen.

Mit der Herstellung wasserlöslicher Salze oder Additionsverbindungen, wie etwa dem Theophyllin-Ethylendiamin (Aminophyllin), ist es inzwischen zwar gelungen, auch parenteral verabreichbare Theophyllin-Ethylendiamin (Aminophyllin), ist es inzwischen zwar gelungen, auch parenteral verabreichbare Theophyllin-Zubereitungen zu erhalten, die aber keine nennenswerten Vergrösserung der therapeutischen Breite und keine Verminderung der oben erwähnten unerwünschten Nebenwirkungen mit sich brachten; zumal das beim Aminophyllin als Lösungsvermittler fungierende Ethylendiamin selbst eine nachteilige Wirkung auf das kardiovaskuläre System ausübt.

Es hat daher nicht an Versuchen gefehlt, durch Strukturvariationen am Theophyllinmolekül zu besser verträglichen Verbindungen mit womöglich stärkerer bronchospasmolytischer Aktivität zu gelangen.

Das einzige synthetische Theophyllinderivat, das eine gewisse therapeutische Anwendung gefunden hat, ist Dyphyllin [7-(2,3-Dihydroxypropyl)-1,3-dimethylxanthin]. Die 7-ständige 2,3-Dihydroxypropylgruppe verleiht diesem Präparat zwar eine gute Wasserlöslichkeit, so dass sich die unerwünschte Anwendung von Lösungsvermittlern bei der parenteralen Applikation erübrigt, und auch die störenden Theophyllin-artigen Nebenwirkungen sind deutlich schwächer ausgeprägt, doch werden diese Vorteile gleichzeitig mit einem drastischen Abfall der bronchospasmolytischen Aktivität gegenüber jener des Theophyllins bezahlt.

In systematischer Fortführung dieser Arbeiten wurden dann unter Beibehaltung des 2,3-Dihydroxypropylrestes in der 7-Stellung des Xanthingerüstes die beiden 1- und 3-ständigen Methylgruppen gegen längeres Alkyl ausgetauscht. Dies führte zu dem in der DE-A-2716402 beschriebenen 7-(2,3-Dihydroxypropyl)-1,3-dipropylxanthin, einer gut wasserlöslichen Verbindung, die fast die bronchospasmolytische Wirkung von Theophyllin erreichen und gleichzeitig eine geringere akute Toxizität sowie weniger nachteilige Nebenwirkungen aufweisen soll. Dennoch hat dieses Präparat bisher keinen Eingang in die Asthmatherapie gefunden. Darüberhinaus ruft es laut vorgenannter Offenlegungsschrift eine wenn auch deutlich schwächere zentrale Stimulierung als Theophyllin hervor, die zu Unruhe und Schlafstörungen führen kann. Demzufolge würden wasserlösliche Xanthinverbindungen, die dem Theophyllin an Wirkungsstärke und therapeutischer Breite überlegen sind und keine nennenswerten Nebenwirkungen insbesondere keine zentrale Erregung auslösen, nach wie vor eine echte Bereicherung der Therapie von obstruktiven Atemwegsleiden darstellen.

Überraschend wurde nun gefunden, dass die bislang nicht untersuchte Verlängerung des Dihydroxypropylrestes unabhängig von dessen Stellung am Xanthingerüst zu Verbindungen führt, die diese strengen therapeutischen Anforderungen erfüllen. Zwar sind in der Literatur bereits zwei derartige Xanthinkörper, nämlich 1-(5,6-Dihydroxyhexyl)-3,7-dimethylxanthin und 1-(4,5-Dihydroxyhexyl)-3,7-dimethylxanthin in der threo- und erythro-Form beschrieben worden (Arzneimittelforschung (Drug Res.) 22, 1144–1151 (1972), und 22, 1492–1495), doch wurden diese Verbindungen lediglich als Metabolite des Vasotherapeutikums Pentoxifyllin isoliert und identifiziert. Dementsprechend finden sich in diesen Publikationen auch keinerlei Angaben über die pharmakologischen Eigenschaften der beiden Verbindungen.

Gegenstand der vorliegenden Erfindung sind somit Arzneimittel, die vicinale Dihydroxyalkylxanthine der allgemeinen Formel (I) (s. Patentanspruch 1) enthalten, worin einer der Reste $R^1$, $R^2$ oder $R^3$ eine Dihydroxyalkylgruppe der Formel $CH_2OH{-}CHOH{-}(CH_2)_n{-}$ oder $CH_3{-}CHOH{-}CHOH{-}(CH_2)_{n-1}{-}$ mit n=2–6 bedeutet und die beiden anderen Reste geradkettige oder verzweigte Alkylgruppen mit bis zu 12 C-Atomen in der Position von $R^1$ und $R^3$ und bis zu 4 C-Atomen in der Position von $R^2$ darstellen, wobei die Summe der C-Atome dieser beiden Alkylsubstituenten höchstens 14 beträgt.

Bevorzugt sind dabei solche Arzneimittel, die Verbindungen der Formel (I) enthalten, in denen $R^1$ oder $R^2$ einen $CH_2OH{-}CHOH{-}(CH_2)_n{-}$ Rest mit n=3 oder 4 darstellt und die beiden Alkylsubstituenten $R^2$ und $R^3$ zusammen 3 bis 6 C-Atome umfassen.

Eine weitere bevorzugte Ausführungsform der Erfindung betrifft Arzneimittel, die solche Verbindungen der Formel (I) enthalten, in denen $R^3$ eine $CH_2OH{-}CHOH{-}(CH_2)_n{-}$Gruppe mit n=2 bis 5 oder eine 4,5-Dihydroxyhexylgruppe bedeutet und die

Summe der C-Atome der beiden Alkylreste $R^1$ und $R^2$ 3 bis 7 beträgt. Unter diesen Arzneimitteln sind wiederum jene mit solchen Verbindungen der Formel (I) besonders bevorzugt, in denen $R^3$ einen 5,6-Dihydroxyhexylrest, wie etwa im 3-Äthyl-7-(5,6-dihydroxyhexyl)-1-propylxanthin, darstellt.

Eine weitere besondere Ausführungsform der Erfindung besteht darin, dass die Verbindungen der Formel (I) nicht per se, sondern in Prodrug-Form, und zwar in Form von Epoxyden bzw. Acetalen verabreicht werden, aus der erst im Organismus die bronchospasmolytisch aktiven Dihydroxyalkylxanthine mit ihren voranstehend definierten Substituenten $R^1$, $R^2$ und $R^3$ durch Biotransformation freigesetzt werden können. Hierfür eignen sich die nachfolgend bei den Herstellungsverfahren als Zwischenprodukte abgehandelten Epoxide gemäss Formel (II) mit dem Strukturelement der Formel (IV) und insbesondere die Acetale gemäss Formel (VII) mit dem Strukturelement der Formel (IX), soweit diese vollständig alkyliert sind.

Gegenstand der Erfindung sind auch neue vicinale Dihydroxyalkylxanthine der Formel (I), wie oben definiert, mit der Massgabe, dass $R^2$ und $R^3$ nicht gleichzeitig Methyl sind, wenn $R^1$ einen 4,5- oder 5,6-Dihydroxyhexylrest darstellt.

Besonders geeignete Verbindungen sind dabei sowohl solche, in denen $R^1$ oder $R^2$ einen $CH_2OH-CHOH-(CH_2)_n$–Rest mit n=3 oder 4 darstellt und die beiden Alkylsubstituenten $R^2$ und $R^3$ bzw. $R^1$ und $R^3$ zusammen 3 bis 6 C-Atome enthalten, als auch jene, worin $R^3$ eine $CH_2OH-CHOH-(CH_2)_n-$ Gruppe mit n=2 bis 5 oder eine 4,5-Dihydroxyhexylgruppe bedeutet und die beiden Alkylreste $R^1$ und $R^2$ zusammen 3 bis 7 C-Atome enthalten. Unter den zuletzt genannten Verbindungen wieder stellen die 7-(5,6-Dihydroxyhexyl)-1,3-dialkylxanthine wie etwa das 3-Äthyl-7-(5,6-dihydroxyhexyl)-1-propylxanthin, besonders bevorzugte Verbindungen gemäss Formel (I) dar.

Gegenstand der Erfindung sind weiterhin Verfahren zur Herstellung der vicinalen Dihydroxyalkylxanthine gemäss Formel (I).

Ein Verfahren besteht beispielsweise darin, dass man Xanthine der Formel (II) (s. Patentanspruch 7 bzw. Patentanspruch 1 für Österreich), in der einer der Reste $R^{1'}$, $R^{2'}$ oder $R^{3'}$ eine Alkenylgruppe der Formel $-(CH_2)_n-CH=CH_2$ (IIIa) oder $-(CH_2)_{n-1}-CH=CH-CH_3$ (IIIb) ist, wobei n jeweils 2 bis 6 ist und die beiden anderen Substituenten Wasserstoff oder wie bei Formel (I) definiertes Alkyl darstellen,

a) mit geeigneten Oxidationsmitteln an der olefinischen Doppelbindung zu neuen Epoxyalkylxanthinen mit dem Strukturelement der Formel

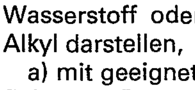 (IVa)

oder 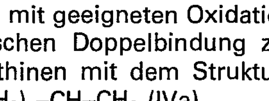

(IVb) umsetzt und deren Oxiranring unter Bildung von Dihydroxyalkylxanthinen mit dem Strukturmerkmal der Formel $-(CH_2)_n-CHOH-CH_2OH$ (Va) bzw. $-(CH_2)_{n-1}-CHOH-CHOH-CH_3$ (Vb) hydrolytisch öffnet oder

b) mit üblichen Oxidationsmitteln an der olefinischen Doppelbindung direkt zu den Dihydroxyalkylxanthinen mit dem durch Formel (Va) bzw. (Vb) gekennzeichneten Strukturelement dihydroxyliert und anschliessend jene der nach a) oder b) erhaltenen Diole, die noch Wasserstoff in den Positionen von $R^{1'}$, $R^{2'}$ und/oder $R^{3'}$ tragen, gegebenenfalls in Gegenwart basischer Mittel oder in Form ihrer Salze mit Alkylierungsmitteln der Formel $R^4$–X (VI), in der X Halogen, vorzugsweise Chlor oder Brom, oder eine Sulfonsäureester- oder Phosphorsäureester-Gruppierung und $R^4$ die für Formel (I) definierten Alkylreste bedeuten, zu den Verbindungen der Formel (I) alkyliert, oder dass man eine Verbindung der Formel (VII) (s. Patentanspruch 7 bzw. Patentanspruch 1 für Österreich), in der höchstens zwei der Substituenten $R^{1''}$ bis $R^{3''}$ für bei Formel (I) definiertes Alkyl stehen und höchstens zwei dieser Reste Wasserstoff bedeuten, gegebenenfalls in Gegenwart basischer Mittel oder in Form ihrer Salze,

c) mit Alkylierungsmitteln der Formel (VIIIa) bzw. (VIIIb) (s. Patentanspruch 7 bzw. Patentanspruch 1 für Österreich) mit jeweils n=2–6, worin $R^5$ und $R^6$ unabhängig voneinander Wasserstoff, niederes Alkyl mit vorzugsweise bis zu 4 C-Atomen, Phenylalkyl mit bis zu 2 C-Atomen im Alkylteil oder gegebenenfalls substituiertes Phenyl und X Halogen, vorzugsweise Chlor oder Brom, oder eine Sulfonsäureester- oder Phosphorsäureester-Gruppierung bedeuten, zu neuen di- oder trialkylierten Xanthinen mit dem Strukturelement der Formel (IXa) der (IXb) (s. Patentanspruch 7 bzw. Patentanspruch 1 für Österreich) umsetzt und deren 1,3-Dioxolanring unter Abspaltung von $R^5$–CO–$R^6$ und Bildung von Dihydroxyalkylxanthinen mit dem Strukturmerkmal der Formel (Va) bzw. (Vb) (s. oben unter a)) hydrolytisch öffnet oder

d) mit Alkylierungsmitteln der Formel (Xa) bzw. (Xb) (s. Patentanspruch 7 bzw. Patentanspruch 1 für Österreich), worin n=2–6 ist und X die angegebene Bedeutung hat, direkt zu den Dihydroxyalkylxanthinen mit dem durch Formel (Va) bzw. (Vb) gekennzeichneten Strukturmerkmal umsetzt und anschliessend die nach c) oder d) erhaltenen Monoalkyldihydroxyalkylxanthine, die noch ein Wasserstoffatom in der Position von $R^{1'''}$, $R^{2'''}$ oder $R^{3'''}$ tragen, gegebenenfalls in Gegenwart basischer Mittel oder in Form ihrer Salze, mit Alkylierungsmitteln der Formel $R^4$–X (VI), in der X und $R^4$ die bei Formel (VI) definierten Bedeutungen haben, zu den Verbindungen der Formel (I) umsetzt oder aber die nach c) hergestellten dialkylierten Xanthine mit dem Strukturelement der Formel (IXa) bzw. (IXb) zuerst mit den Verbindungen der Formel $R^4$–X (VI) alkyliert und dann den Dioxolanring unter Bildung der Dihydroxyalkylxanthine gemäss Formel (I) hydrolytisch spaltet.

Gegenstand der Erfindung sind schliesslich auch die neuen Zwischenprodukte der Formel (XI) (s. Patentanspruch 8), in der einer der Reste $R^1$, $R^2$ und $R^3$ eine Gruppe der Formel (IVa) bzw. (IVb) oder (IXa) bzw. (IXb) ist (s. Patentanspruch 8), wobei n=2 bis 6, $R^5$ und $R^6$ unabhängig voneinan-

der Wasserstoff, Methyl, Phenylalkyl mit bis zu 2 C-Atomen im Alkylteil oder Phenyl sind, die übrigen der Reste $R^1$, $R^2$ und $R^3$ unabhängig voneinander Wasserstoff oder Alkyl mit 1 bis 12 C-Atomen in den Positionen $R^1$ und $R^3$ und mit 1 bis 4 C-Atomen in der Position $R^2$ bedeuten und die Summe der Kohlenstoffatome dieser beiden Reste höchstens 14 beträgt.

Die bei den Ausführungsformen a) und b) als Ausgangsstoffe benutzten Alkenyl-, Monoalkyl- und Dialkylalkenylxanthine der Formel (II) sind unter anderem aus den DE-A-2714953 und DE-A-2836147 bekannt.

Als geeignete Oxidationsmittel für die Epoxidation der olefinischen Seitenkette gemäss Formel (III) kommen beispielsweise Chrom-(VI)-oxid, vorzugsweise in Essigsäureanhydrid und schwefelkohlenstoff bzw. Tetrachlorkohlenstoff; oder peroxidgruppenhaltige Verbindungen wie Kaliumperoxomonosulfat in Gegenwart von Ketonen, bevorzugt Aceton, in homogener Phase oder aber in einem Zweiphasen-System unter Phasentransferkatalyse; Peroxyborane, die man vorteilhafterweise aus Borsäure oder deren Derivaten und Hydroperoxiden in situ erzeugt; Triphenylsilylhydroperoxid; Wasserstoffperoxid in Gegenwart von Coreaktanden, wie beispielsweise aliphatischen oder aromatischen Carbonsäurenitrilen (z.B. Aceto- oder Benzonitril), gegebenenfalls substituiertem Cyanamid oder Isocyanaten (z.B. Phenylisocyanat); Wasserstoffperoxid oder Alkyl- bzw. Aralkylhydroperoxide, wie etwa tert.-Butylhydroperoxid, 1-Phenylethylhydroperoxid und Cumolhydroperoxid, in Gegenwart entweder basischer Mittel oder bevorzugt spezieller Katalysatoren, wie beispielsweise Wolframsäure, Vanadium-(V)oxid, Molybdänhexacarbonyl und Vanadium- oder Molybdänacetylacetonate; und insbesondere Percarbonsäuren, wie z.B. Perameisen-, Peressig-, Trifluorperessig-, Monopermalein-, Monoperbernstein-, Perbenzoe-, 4-Nitroperbenzoe- und vorzugsweise 3-Chlorperbenzoe- und Monoperphthalsäure, in Frage.

Die Epoxidation mit Hilfe von Percarbonsäuren (Prileschajew-Reaktion) wird zweckmässig in einem gegenüber den Reaktionsteilnehmern inerten Lösungs- oder Verteilungsmittel durchgeführt, das erfahrungsgemäss einen beachtlichen Einfluss auf die Reaktionsgeschwindigkeit ausübt. Da Lösungsmittel, die mit den Percarbonsäuren Wasserstoffbrückenbindungen bilden können, im allgemeinen die Reaktionsgeschwindigkeit herabsetzen, sind aromatische Kohlenwasserstoffe, wie Benzol oder Toluol, und halogenierte Kohlenwasserstoffe, wie Dichlormethan, Chloroform oder Tetrachlormethan, gegenüber Äthern, wie Diäthyläther, Dioxan, Tetrahydrofuran oder Äthylenglykoldimethyläther, Alkoholen, Estern und Carbonsäuren häufig bevorzugt. Die Umsetzung erfolgt üblicherweise bei Temperaturen zwischen −10° und +40°C, vorzugsweise bei Raumtemperatur, wobei die Reaktionszeit von wenigen Minuten bis zu mehreren Stunden reichen kann.

Gewöhnlich setzt man Percarbonsäuren in isolierter Form zur Reaktion ein, sie können aber auch beispielsweise aus der entsprechenden Carbonsäure und Wasserstoffperoxid im Reaktionsansatz in situ erzeugt werden.

Bei der Verwendung von Persäuren starker Carbonsäuren, wie etwa der Trifluorperessigsäure, empfiehlt es sich, die Säurekonzentration durch Arbeiten im heterogenen System oder durch Zusatz von Puffersubstanzen, wie Natriumcarbonat, Natriumhydrogencarbonat oder Dinatriumhydrogenphosphat, herabzusetzen, um unerwünschte Folgereaktionen der bei der Reaktion entstehenden Carbonsäure mit dem primär gebildeten Epoxid zu unterdrücken.

Die Epoxyalkylxanthine gemäss Formel (II) mit dem Strukturmerkmal der Formel (IVa) bzw. (IVb) können aber auch durch basenkatalysierte Dehydrohalogenierung von entsprechenden Halogenhydrinen erhalten werden, die sich ihrerseits z.B. durch Anlagerung von unterhalogenigen Säuren, wie etwa unterchloriger Säure, an die olefinische Doppelbindung der Alkenylxanthine gemäss den Formeln (II) und (III) gewinnen lassen. Auch die Umsetzung dieser Olefine mit N-Halogensuccinimiden, wie N-Bromsuccinimid, oder Chloramin T in Wasser oder wasserhaltigen Lösungsmittelgemischen führt zu den Halogenhydrinen. Als basische Dehydrohalogenierungsmittel verwendet man gewöhnlich Alkali- oder Erdalkalihydroxide oder -carbonate, vorzugsweise Natrium-, Kalium- oder Calciumhydroxid oder Natrium- oder Kaliumcarbonat, aber auch organische Basen oder andere Oxirane, wie Ethylenoxid oder 1,2-Epoxypropan, können mit Erfolg eingesetzt werden.

Die Epoxyalkylxanthine können entweder in reiner Form isoliert oder aber als Rohprodukte weiterverarbeitet werden.

Die hydrolytische Spaltung der Epoxyalkylxanthine zu den vicinalen Diolen mit dem Strukturelement der Formel (Va) bzw. (Vb) wird in wässrigem Medium, dem man gegebenenfalls zur Lösungsverbesserung ein mit Wasser mischbares organisches Lösungsmittel, beispielsweise Tetrahydrofuran, Dioxan oder Ethylenglykoldimethylether, zusetzt, vorteilhaft in Gegenwart saurer Katalysatoren, vorzugsweise schwach nucleophiler Säuren, wie Schwefel-, Perchlor- oder p-Toluolsulfonsäure oder auch stark saurer Kationenaustauscherharze (z.B. Nafion-H), bei Temperaturen zwischen 20° und 100°C, bevorzugt aber unter mehrstündigem Rühren bei Raumtemperatur, durchgeführt. Aber auch unter neutralen oder alkalischen Bedingungen ist die Oxiranringöffnung prinzipiell möglich.

Übliche Oxidationsmittel für die direkte vicinale Dihydroxylierung der Alkenylxanthine gemäss Formeln (II) und (III) zu den durch das Strukturmerkmal der Formel (Va) bzw. (Vb) gekennzeichneten Dihydroxyalkylxanthinen stellen beispielsweise Wasserstoffperoxid in Gegenwart von Ameisensäure oder Eisessig, Chromylchlorid, Kaliumpermanganat, Triphenylmethylphosphoniumpermanganat, Jod in Gegenwart von Silber- oder Thallium(I)carboxylaten, wie z.B. Thallium-(I)acetat, Selendioxid, Molybdän(VI)oxid und insbesondere Osmiumtetroxid dar.

Bei der Verwendung von Osmiumtetroxid als Oxidationsmittel kann das Reagens entweder in stöchiometrischer Menge oder aber in katalytischen Mengen unter Zusatz eines sekundären Oxidationsmittels, das das Osmiumtetroxid aus den primär entstehenden cyclischen Estern unter oxidativer Hydrolyse zu den Diolen regeneriert, eingesetzt werden.

Bei der nicht-katalytischen Dihydroxylierung der olefinischen Doppelbindung mit stöchiometrischen Mengen an Osmiumtetroxid arbeitet man zweckmässig in nicht reduzierend wirkenden Lösungsmitteln, vorzugsweise Äthern, wie Diäthyläther, Tetrahydrofuran und Dioxan, oder Kohlenwasserstoffen, wie Benzol, Cyclopentan oder -hexan, gegebenenfalls unter Zusatz eines tertiären Amins, wie vor allem Pyridin aber auch Chinolin, Isochinolin, 3- oder 4-Picolin, bei Temperaturen zwischen 0°C und dem Siedepunkt des jeweiligen Lösungsmittels, vorzugsweise um Raumtemperatur, wobei die Reaktionszeiten von einigen Minuten bis zu mehreren Stunden betragen können. Anschliessend werden die hierbei intermediär entstehenden Osmium(VI)ester-Komplexe vorteilhafterweise reduktiv hydrolysiert, wobei sich insbesondere der Einsatz von Natrium- oder Kaliumsulfit oder -hydrogensulfit, Schwefelwasserstoff, Lithiumaluminiumhydrid oder auch Brenzcatechin oder alkalischer Mannitol-Lösung in wässrigem oder wässrig-alkoholischem Medium bewährt. Aber auch die oxidative Hydrolyse der Komplexe ist möglich; allerdingt empfiehlt es sich in diesem Falle die Dihydroxylierung von vornherein mit katalytischen Mengen an Osmiumtetroxid in Gegenwart sekundärer Oxidationsmittel, wie beispielsweise Hydrogenperoxid, Metallchloraten (z.B. Natrium- oder Kalium- und insbesondere Silber- oder Bariumchlorat), Natriumperchlorat, Sauerstoff, Natriumperjodat oder -hypochlorit und vor allem tert.-Butylhydroperoxid oder Trialkylamin-N-oxiden (z.B. N-Methylmorpholin-N-oxid, Trimethyl- oder Triäthylamin-N-oxid), durchzuführen.

Die Alkylierung der Dihydroxyalkylxanthine mit den Verbindungen der Formel (VI) erfolgt gewöhnlich in einem gegenüber den Reaktionsteilnehmern inerten Verteilungs- oder Lösungsmittel. Als solche kommen vor allem dipolare, aprotische Solventien, beispielsweise Formamid, Dimethylformamid, Dimethylacetamid, N-Methylpyrrolidon, Tetramethylharnstoff, Hexamethylphosphorsäuretriamid, Dimethylsulfoxid, Aceton oder Butanon, in Frage; es können aber auch Alkohole, wie Methanol, Äthylenglykol und dessen Äther, Äthanol, Propanol, Isopropanol und die verschiedenen Butanole; Kohlenwasserstoffe, wie Benzol, Toluol oder Xylole; halogenierte Kohlenwasserstoffe, wie Dichlormethan oder Chloroform; Pyridin sowie Mischungen der genannten Lösungsmittel oder deren Gemische mit Wasser Verwendung finden.

Die Umsetzungen werden zweckmässig in Gegenwart eines basischen Kondensationsmittels durchgeführt. Hierfür eignen sich beispielsweise Alkali- oder Erdalkalihydroxide, -carbonate, -hydride, Alkoholate oder organische Basen, wie Trialkylamine (z.B. Triäthyl- oder Tributylamin), quartäre Ammonium- oder Phosphoniumhydroxide und vernetzte Harze mit fixierten, gegebenenfalls substituierten Ammonium- oder Phosphoniumgruppen.

Die Xanthinderivate können aber auch unmittelbar in Form ihrer gesondert hergestellten Salze, etwa der Alkali-, Erdalkali- oder gegebenenfalls substituierten Ammonium- oder Phosphoniumsalze, in die Alkylierungsreaktion eingesetzt werden. Weiterhin lassen sich die Dihydroxyalkylxanthine und deren monoalkylierten Derivate sowohl in Gegenwart der vorgenannten anorganischen Kondensationsmittel als auch in Form ihrer Alkali- oder Erdalkalisalze unter Mithilfe von sogenannten Phasentransferkatalysatoren, beispielsweise tertiären Aminen, quartären Ammonium- oder Phosphoniumsalzen oder auch Kronenethern, bevorzugt in einem zweiphasigen System unter den Bedingungen einer Phasentransferkatalyse bequem alkylieren.

Bei der Einführung der Alkylreste nach den voranstehend beschriebenen Verfahrensweisen arbeitet man im allgemeinen bei einer Reaktionstemperatur zwischen 0°C und dem Siedepunkt des jeweils verwendeten Reaktionsmediums, vorzugsweise zwischen 20° und 130°C, gegebenenfalls bei erhöhtem oder vermindertem Druck, aber gewöhnlich bei Atmosphärendruck, wobei die Reaktionszeit von weniger als eine Stunde bis zu mehreren Stunden betragen kann.

Bei jenen Dihydroxyalkylxanthinen, in die noch zwei Alkylreste einzuführen sind, können hierbei entweder nacheinander gleiche oder verschiedene Substituenten oder auch zwei gleichartige Alkylgruppen ohne Isolierung von Zwischenprodukten in einer Eintopfreaktion mit dem Xanthingerüst verknüpft werden.

Die bei den Ausführungsformen c) und d) als Ausgangsstoffe verwendeten Monoalkyl- oder Dialkylxanthine der Formel (VII) und Alkylierungsmittel der Formeln (VIIIa) bzw. (VIIIb) und (Xa) bzw. (Xb) sind grösstenteils bekannt oder lassen sich nach literaturbekannten Methoden leicht herstellen.

So können die Verbindungen der Formel (VIII) beispielsweise aus den Triolen der Formel $HO-(CH_2)_n-CHOH-CHOH-R^4$ (XI) durch Umsetzung der beiden vicinalen Hydroxylgruppen mit Aldehyden oder Ketonen oder auch deren Acetalen unter Protonenkatalyse und anschliessenden Austausch der isolierten, endständigen Hydroxylfunktion gegen Halogen mittels anorganischer Säurehalogenide oder durch Veresterung mit Sulfonsäure- oder Phosphonsäurehalogeniden bzw. -anhydriden vorteilhaft in Gegenwart basischer Mittel erhalten werden, aus denen durch saure Hydrolyse des 1,3-Dioxolanringes wiederum die Verbindungen der Formel (X) darstellbar sind. Auch die Alkenylhalogenide der Formel $Hal-(CH_2)_n-CH{=}\ CH-R^4$ (XII) können als Ausgangsstoffe für die Herstellung von Verbindungen der Formeln (VIIIa) bzw. (VIIIb) und (Xa) bzw. (Xb) dienen, indem man sie – wie für die Alkenylxanthine beschrieben – entweder an der olefinischen Doppelbindung epoxidiert und anschliessend den Oxiranring sauer hydrolysiert oder aber in einstufiger Reaktion direkt zu den Dihydroxyalkylhalogeniden der Formel (Xa) bzw. (Xb) oxidiert und diese gegebenenfalls mit Aldehyden oder Ketonen oder deren Acetalen in 1,3-Dioxolane der Formel (VIIIa) bzw. (VIIIb) überführt.

Die Umsetzungen der Xanthinderivate mit den Alkylierungsmitteln der Formeln (VI), (VIIIa) bzw. (VIIIb) und (Xa) bzw. (Xb) erfolgen zweckmässig unter den für die Alkylierung der Dihydroxyalkyl- und Monoalkyldihydroxyalkylxanthine mit den Verbindungen der Formel (VI) bereits ausführlich beschriebenen Reaktionsbedingungen. Verwendet man allerdings die Verbindungen der Formel (Xa) bzw. (Xb) zur Einführung des Dihydroxyalkylrestes, so sind unter ihnen jene bevorzugt, deren Alkylkette insgesamt in Formel (Xa) 6 bis 8 C-Atome oder aber in Formel (Xb) 7 oder 8 C-Atome aufweist, da die kürzerkettigen Diole der Formel (Xa) bzw. (Xb) speziell unter den alkalischen Bedingungen der Alkylierungsreaktion zur Bildung von Tetrahydrofuranderivaten neigen, was zu einer merklichen Schmälerung der Ausbeute an erwünschtem Alkylierungsprodukt führen kann.

Die hydrolytische Spaltung des 1,3-Dioxolanringes in den Xanthinen der Formel (VII) mit dem Strukturelement der Formel (IXa) bzw. (IXb) zu den durch das Strukturmerkmal der Formel (Va) bzw. (Vb) gekennzeichneten Dihydroxyalkylxanthinen wird normalerweise in wässrigem Medium, gegebenenfalls unter Zusatz eines Lösungsvermittlers, wie Tetrahydrofuran, Dioxan oder Äthylenglykoldimethyläther, vorteilhaft in Gegenwart von Säuren, beispielsweise Ameisen-, Oxal-, Wein-, Citronen-, Schwefel-, Perchlor-, Phosphor- oder p-Toluolsulfonsäure, oder eines sauren Ionenaustauschers (z.B. Nafion-H) bei Temperaturen zwischen 20°C und dem Siedepunkt des Reaktionsgemisches, vorzugsweise 50° und 100°C, durchgeführt, wobei die Reaktionszeit von mehreren Minuten bis zu wenigen Stunden betragen kann. Auch feuchtes Silicagel mit einem Wassergehalt bis zu 10% stellt ein brauchbares Reagens für die Deacetalisierung dar, wobei bevorzugt in gegebenenfalls halogenierten Kohlenwasserstoffen, wie Benzol, Toluol, Dichlormethan oder Chloroform, bei Raumtemperatur gearbeitet wird.

Eine weitere Darstellungsmethode für die Xanthine der Formel (VII) mit dem den Dioxolanring enthaltenden Strukturelement der Formel (IXa) bzw. (IXb) besteht in der Addition von Carbonylverbindungen der Formel $R^6$–CO–$R^7$ an den Oxiranring der Epoxyalkylxanthine gemäss Formel (II) mit dem Strukturmerkmal der Formel (IVa) bzw. (IVb). Diese Umsetzung wird vorteilhaft in Gegenwart von sauren Katalysatoren, vorzugsweise Lewis-Säuren wie Bortrifluorid, Zink(II)-chlorid, Zinn(IV)chlorid oder auch Kupfer(II)sulfat, bei Temperaturen zwischen 0° und 60°C ausgeführt. Aber auch quartäre Ammoniumsalze, beispielsweise Tetraethylammoniumhalogenide, vermögen die Additionsreaktion zu den cyclischen Acetalen zu katalysieren.

Die vicinalen Dihydroxyalkylxanthine der Formel (I) besitzen in Abhängigkeit von der Stellung der beiden Hydroxylgruppen in der Seitenkette gemäss Formel (Va) bzw. (Vb) entweder ein oder zwei asymmetrische C-Atome und können somit in stereoisomeren Formen vorliegen. Die Erfindung betrifft daher sowohl die reinen stereoisomeren Verbindungen als auch deren Gemische.

Die erfindungsgemässen Arzneimittel können oral, rektal, parenteral oder als Aerosol verabreicht werden.

Geeignete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Aerosole, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmackstoffe, Süssungsmittel oder Lösungsvermittler, Verwendung finden. Als häufig verwendete Hilfsstoffe seien z.B. Laktose, Mannit und andere Zucker, Talkum, Milcheiweiss, Gelatine, Stärke, Cellulose und ihre Derivate, tierische und pflanzliche Öle, Polyäthylenglykole und Lösungsmittel, wie etwa steriles Wasser, genannt.

Vorzugsweise werden die Präparate in Dosierungseinheiten hergestellt und verabreicht, wobei jede Einheit eine bestimmte Dosis an aktiver Substanz gemäss Formel (I) enthält. Bei festen Dosierungseinheiten, wie Tabletten, Kapseln und Suppositorien, kann diese Dosis bis zu 1000 mg, bevorzugt jedoch 50 bis 300 mg, und bei Injektionslösungen in Ampullenform bis zu 200 mg, vorzugsweise aber 20 bis 100 mg, betragen.

Für die Behandlung eines an bronchialen Obstruktionen leidenden erwachsenen Patienten sind – je nach Wirksamkeit der Verbindungen gemäss Formel (I) am Menschen – Tagesdosen von 100 bis 500 mg Wirkstoff, vorzugsweise 200 bis 300 mg, bei oraler Verabreichung und von 20 bis 150 mg, bevorzugt 40 bis 80 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schliesslich können die Xanthinderivate der Formel (I) bei der Herstellung der vorgenannten galenischen Zubereitungsformen auch zusammen mit anderen geeigneten Wirkstoffen, beispielsweise Antiallergika, Antitussiva, Sekretolytika, Sedativa, peripheren Vasotherapeutika, Antihistaminika und auch anderen Bronchospasmolytika, wie $\beta_2$-Sympathomimetika oder Parasympatholytika formuliert werden.

Beispiele

Die Struktur aller nachstehend beschriebenen Verbindungen wurde durch Elementaranalyse und IR- sowie $^1$H-NMR-Spektren gesichert.

Beispiel 1
3-Ethyl-7-(5,6-dihydroxyhexyl)-1-propylxanthin

a) 1-Hydroxy-5,6-isopropylidendioxy-hexan

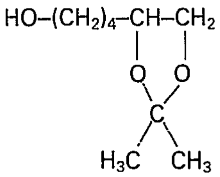

Zur Mischung aus 830g 1,2,6-Hexantriol (97%ig) und 828ml 2,2-Dimethoxypropan (98%ig) werden bei Raumtemperatur während 5 Minuten tropfenweise 3ml 98%ige Schwefelsäure hinzugefügt. Nach einstündigem Nachrühren bei 25°C setzt man 30g Kaliumcarbonat zu, rührt eine weitere Stunde und führt anschliessend eine Vakuumdestillation über eine 10cm Füllkörperkolonne durch.
Ausbeute: 897g (86% der Theorie)
Siedepunkt (0,5m bar) 83–87°C
Brechungsindex $n_D^{20}$ = 1,4452

b) 1-Chlor-5,6-isopropylidendioxy-hexan

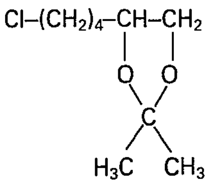

Zur Lösung von 176,4g 1-Hydroxy-5,6-isopropylidendioxy-hexan und 155ml Triethylamin in 1300ml Toluol werden unter Rühren bei 5–7°C Innentemperatur 77ml Thionylchlorid innerhalb 3 Stunden zugetropft. Nach halbstündigem Nachrühren bei 20–25°C erhitzt man bis zur Beendigung der $SO_2$-Entwicklung auf 70°C (ca. 4 Stunden). Danach wird abgekühlt und vom ausgefallenen Niederschlag abgenutscht. Nach Nachwaschen mit 100ml Toluol werden die Toluolphasen vereinigt, neutralgewaschen, getrocknet und unter vermindertem Druck eingeengt. Den Rückstand versetzt man mit 15g Kaliumcarbonat und destilliert im Vakuum über eine Füllkörperkolonne.
Ausbeute: 149,6g (77,6% der Theorie)
Siedepunkt (0,15m bar) 48–50°C
Brechungsindex $n_D^{18}$ = 1,4482

c) 3-Ethyl-7-(5,6-isopropylidendioxy-hexyl)-xanthin

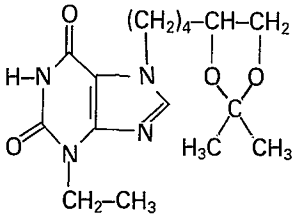

Das Gemisch aus 360,4g 3-Ethylxanthin, 409,2g 1-Chlor-5,6-isopropylidendioxy-hexan und 284,7g Kaliumcarbonat in 3 l Dimethylformamid wird 2 Stunden unter Rühren bei 100°C erhitzt. Nach Einengen der Suspension bei vermindertem Druck nimmt man mit 1,1 l 2n Natronlauge auf und extrahiert gründlich mit Methylenchlorid. Die gesammelten Methylenchloridphasen werden nochmals mit 2n Natronlauge gewaschen, mit Wasser neutralgewaschen, getrocknet und bei vermindertem Druck eingeengt.

Man erhält 94,5g rohes 3-Ethyl-1,7-bis-(5,6-isopropylidendioxy-hexyl)-xanthin als Nebenprodukt. Die vereinigten wässrig-natronalkalischen Phasen werden unter Rühren bei Raumtemperatur tropfenweise mit 33%iger Schwefelsäure bis zum Erreichen von pH=10 versetzt. Der Niederschlag wird abgenutscht, neutralgewaschen und bei 100°C im Vakuum getrocknet.
Ausbeute: 508g (75,5% der Theorie)
Schmelzpunkt: 123–124°C
$C_{16}H_{24}N_4O_4$ (MG=336,4)
Analyse: Berechnet: C 57,13% H 7,19% N 16,66%
Gefunden: C 56,92% H 7,21% N 16,68%

d) 3-Ethyl-7-(5,6-dihydroxyhexyl)-1-propylxanthin

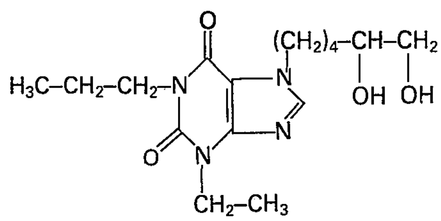

336,4g 3-Ethyl-7-(5,6-isopropylidendioxy-hexyl)-xanthin, 151g 1-Brompropan und 138g Kaliumcarbonat werden 48 Stunden bei 70°C Innentemperatur in 1,5 l Dimethylformamid gerührt. Nach Entfernen des Lösungsmittels bei vermindertem Druck nimmt man mit Methylenchlorid auf, wäscht mit verdünnter Natronlauge und engt die neutralisierte und getrocknete Methylenchloridphase ein. Der Rückstand wird in 1 l Schwefelsäure beim pH-Wert=0,5 2 Stunden auf 100°C erhitzt. Nach dem Abkühlen neutralisiert man, engt bei vermindertem Druck ein und nimmt den Rückstand mit Methylenchlorid auf. Die Methylenchloridphase wird mit verdünnter Natronlauge und mit Wasser gewaschen, getrocknet und bei vermindertem Druck eingeengt.

Das Rohprodukt wird aus Methylenchlorid/Diethylether umkristallisiert.
Ausbeute: 259g (76,5% der Theorie)
Schmelzpunkt: 96–98°C
$C_{16}H_{26}N_4O_4$ (MG=338,4)
Analyse: Berechnet: C 56,79% H 7,74% N 16,56%
Gefunden: C 56,86% H 7,56% N 16,60%

Beispiel 2
1,3-Diethyl-7-(5,6-dihydroxyhexyl)-xanthin

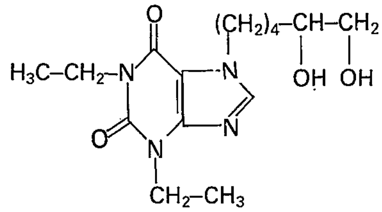

A) Das Gemisch aus 62,5g 1,3-Diethylxanthin, 62,7g 1-Chlor-5,6-isopropylidendioxy-hexan, 42,7g Kaliumcarbonat und 450ml Dimethylformamid wird 10 Stunden bei 120°C gerührt. Nach Einengen bei vermindertem Druck nimmt man mit 300ml 1n Natronlauge auf und extrahiert mit Methylenchlorid. Die Methylenchloridphase wird mit verdünnter Natronlauge nachgewaschen, neutralgewaschen, getrocknet und bei vermindertem Druck eingeengt. Das Rohprodukt destilliert man bei 0,027 mbar und 130–150°C Badtemperatur in einem Dünnschichtverdampfer und erhält 107,2g 1,3-Diethyl-7-(5,6-isopropylidendioxyhexyl)-xanthin.

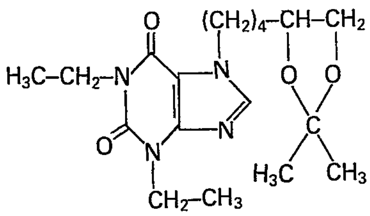

Dieses wird in 1,4l Methanol und 350ml Wasser aufgenommen und nach Zugabe von 1,5ml Perchlorsäure (70%ig) 1 Stunden bei 70°C gerührt. Nach Abkühlen auf Raumtemperatur neutralisiert man mit Natriumbicarbonatlösung und engt bei vermindertem Druck zur Trockne ein. Der Rückstand wird mit 1,5l Methylenchlorid extrahiert und der Extrakt eingeengt. Man erhält 92,5g (95% der Theorie) Rohprodukt, das nach zweimaliger Umkristallisation aus Methylenchlorid/Diethylether (Volumenverhältnis 2:3) dünnschichtchromatographisch reines 1,3-Diethyl-7-(5,6-dihydroxyhexyl)-xanthin ergibt.
Ausbeute: 81,7g (83,9% der Theorie)
Schmelzpunkt: 94–95°C
$C_{15}H_{24}N_4O_4$ (MG=324,4)
Analyse: Berechnet: C 55,54% H 7,46% N 17,27%
Gefunden: C 55,52% H 7,52% N 17,02%

B) Das obige Produkt wird auch durch Hydrolyse von 1,3-Diethyl-7-(5,6-epoxyhexyl)-xanthin (Schmelzpunkt: 58–59°C)

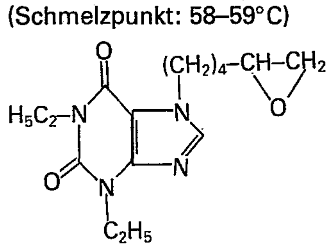

analog Beispiel 3 erhalten.

C) Zu derselben Verbindung gelangt man ausserdem durch Hydroxylierung von 1,3-Diethyl-7-(5-hexenyl)-xanthin

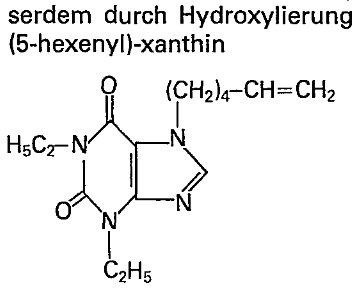

mit Osmiumtetroxid: Zu 0,65g Osmiumtetroxid in 11ml Diethylether werden bei Raumtemperatur unter Rühren in 5 Minuten 0,73g 1,3-Diethyl-7-(5-hexenyl)-xanthin in 11ml Diethylether hinzugetropft.

Nach Stehen über Nacht wird vom Niederschlag (1,2g) abgesaugt. Dieser wird im Gemisch mit 55ml Wasser, 15ml Ethanol und 11,2g Natriumsulfit-heptahydrat 3 Stunden unter Rückfluss gerührt. Nach dem Abkühlen trennt man von entstandenen Niederschlag ab und extrahiert das Filtrat mit Methylenchlorid. Nach Trocknen und Einengen der gesammelten Methylenchloridphasen bei vermindertem Druck erhält man 0,6g Rohprodukt, das nach Umkristallisation aus Diethylether das 1,3-Diethyl-7-(5,6-dihydroxyhexyl)-xanthin als Monohydrat ergibt.
Ausbeute: 0,47g (57,7% der Theorie)
Schmelzpunkt: 77–78°C (Monohydrat)
$C_{15}H_{24}N_4O_4 \cdot H_2O$ (MG=342,4)
Analyse: Berechnet: C 52,62% H 7,65% N 16,36%
Gefunden: C 52,48% H 7,69% N 16,20%

Beispiel 3
1,3-Dimethyl-7-(5,6-dihydroxyhexyl)-xanthin

a) 1,3-Dimethyl-7-(5,6-epoxyhexyl)-xanthin

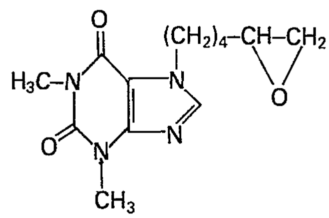

Die Lösung von 31 g 1,3-Dimethyl-7-(5-hexenyl)-xanthin und 34,9 g m-Chlorperbenzoesäure (70%ig) in 700 ml Chloroform wird 48 Stunden bei Raumtemperatur gerührt. Man schüttelt mit 10%iger Natriumdithionitlösung, bis der Test mit Jod-Stärkepapier negativ ausfällt, wäscht mit Natriumbicarbonatlösung und Wasser neutral, trocknet und engt bei vermindertem Druck ein.

Die Reinigung des Rohproduktes erfolgt durch Säulenchromatographie an Kieselgel (Laufmittel: Methylenchlorid/Aceton, Volumenverhältnis 7/3) und durch Umkristallisation aus Petrolether.
Ausbeute: 20,8g (63,2% der Theorie)
Schmelzpunkt: 59–60°C
$C_{13}H_{18}N_4O_3$ (MG = 278,3)
Analyse: Berechnet: C 56,10% H 6,52% N 20,13%
Gefunden: C 55,87% H 6,51% N 19,91%

b) 1,3-Dimethyl-7-(5,6-dihydroxyhexyl)-xanthin

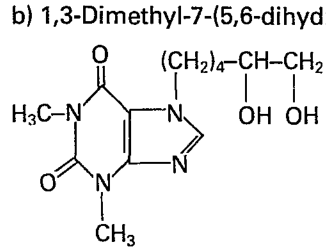

Zur Lösung von 3,6g 1,3-Dimethyl-7-(5,6-epoxyhexyl)-xantin in 280 ml Gemisch aus Ethylenglykoldimethylether und Wasser (Volumenverhältnis

3:2) tropft man unter Rühren bei Raumtemperatur in 5 Minuten 0,24 ml Perchlorsäure (70%ig). Nach 16-stündigem Rühren bei Raumtemperatur wird mit Natriumbicarbonatlösung neutralisiert und bei vermindertem Druck eingeengt. Der Rückstand wird durch Säulenchromatographie an Kieselgel (Laufmittel: Chloroform/Ethanol, Volumenverhältnis 8/2) und Umkristallisation aus Essigester gereinigt.
Ausbeute: 3g (78,3% der Theorie)
Schmelzpunkt: 98–100°C
$C_{13}H_{20}N_4O_4$ (MG = 296,3)
Analyse: Berechnet: C 52,69% H 6,80% N 18,91%
Gefunden: C 52,39% H 6,72% N 18,83%

Sowohl die Umsetzung von 1,3-Dimethyl-7-(5-hexenyl)-xanthin mit Osmiumtetroxid analog Beispiel 2C) als auch die Alkylierung von 1,3-dimethylxanthin mit 1-Chlor-5,6-isopropylidendioxy-hexan und anschliessende saure Hydrolyse des Dioxolanringes gemäss Beispiel 1 bzw. 2 A) führen zu derselben Verbindung.

Beispiel 4
1,3-Dibutyl-7-(3,4-dihydroxybutyl)-xanthin

a) 1,3-Dibuthyl-7-(3,4-epoxybutyl)-xanthin

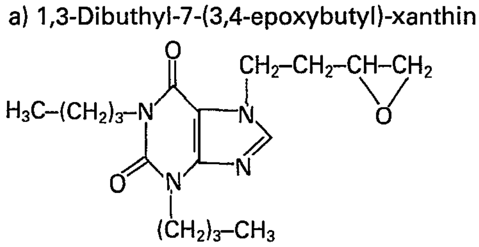

Man versetzt die Lösung von 42,7g 1,3-Dibutyl-7-(3-butenyl)-xanthin in 900 ml Chloroform innerhalb 15 Minuten unter Rühren mit 39,4g m-Chlorperbenzoesäure (70%ig). Nach 27-stündigem Rühren bei Raumtemperatur wird mit 10%iger Natriumdithionit-Lösung, gesättigter Natriumbicarbonatlösung und Wasser gewaschen, getrocknet und bei vermindertem Druck eingeengt. Der Rückstand wird an Kieselgel mit Methylenchlorid/Aceton-Gemisch (7:3, v:v) auf einer Säule chromatographiert und aus Petrolether umkristallisiert.
Ausbeute: 19g (42,4% der Theorie)
Schmelzpunkt: 52–53°C
$C_{17}H_{26}N_4O_3$ (MG = 334,4)
Analyse: Berechnet: C 61,06% H 7,84% N 16,75%
Gefunden: C 61,01% H 7,89% N 16,74%

b) 1,3-Dibutyl-7-(3,4-dihydroxybutyl)-xanthin

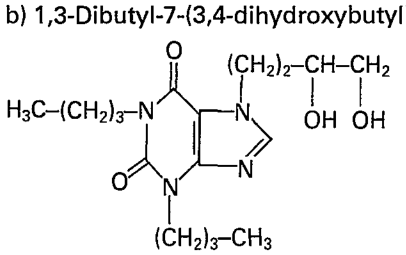

Zu der Lösung von 7g 1,3-Dibutyl-7-(3,4-epoxybutyl)-xanthin in 300 ml eines Gemisches aus Ethylenglykoldimethylether/Wasser (Volumenverhältnis 3:2) tropft man bei Raumtemperatur unter Rühren in 5 Minuten 0,4 ml Perchlorsäure (70%ig). Nach 45 Stunden Rühren bei Raumtemperatur wird mit Natriumcarbonat neutralisiert und die Lösung eingedampft. Der Rückstand wird mit Methylenchlorid aufgenommen und durch Säulenchromatographie an Kieselgel mit dem Gemisch von Chloroform/Ethanol (Volumenverhältnis 8:2) als Laufmittel und durch Umkristallisation aus Methylenchlorid/Petrolether gereinigt.
Ausbeute: 4,8g (65% der Theorie)
Schmelzpunkt: 92–93°C
$C_{17}H_{28}N_4O_4$ (MG = 352,4)
Analyse: Berechnet: C 57,94% H 8,00% N 15,90%
Gefunden: C 58,06% H 8,06% N 15,77%

Dieses Diol lässt sich alternativ durch einstufige Oxidation von 1,3-Dibutyl-7-(3-butenyl)-xanthin mit Osmiumtetroxid analog Beispiel 2 C oder durch Alkylierung von 1,3-Dibutylxanthin mit den literaturbekannten 1-Halogen-3,4-isopropylidendioxy-butanen (z.B. Tetrahedron 34 (1978), S. 2873–2878) und nachfolgende saure Hydrolyse des Dioxolanringes analog Beispiel 1 gewinnen.

Beispiel 5
1,3-Diethyl-7-(6,7-dihydroxyheptyl)-xanthin

a) 1-Brom-6,7-epoxyheptan

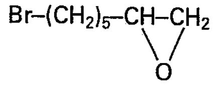

Zu 50,9g m-Chlorperbenzoesäure (85%ig) in 300 ml Methylenchlorid werden unter Rühren und Spülen mit Stickstoff bei Raumtemperatur 37,3g 1-Brom-6-hepten innerhalb von 40 Minuten zugetropft.

Nach Stehen über Nacht wird vom Niederschlag abgesaugt und das Filtrat mit 10%iger $Na_2S_2O_4$-Lösung, mit gesättigter Natriumbicarbonatlösung und mit Wasser gewaschen und (nach Trocknen) unter vermindertem Druck eingeengt. Man erhält 42,2g rohes 1-Brom-6,7-epoxyheptan.

b) 1-Brom-6,7-dihydroxyheptan

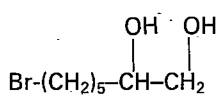

42g 1-Brom-6,7-epoxyheptan werden bei Raumtemperatur in das Gemisch von 400 ml Tetrahydrofuran und 235 ml Wasser, das mit Perchlorsäure auf pH 2 eingestellt ist, eingetragen. Nach 8 Stunden Rühren bei Raumtemperatur neutralisiert man, engt bei vermindertem Druck ein und extrahiert den Rückstand mit Methylenchlorid. Nach Entfernen des Lösungsmittels erhält man 41,5g rohes 1-Brom-6,7-dihydroxyheptan.

c) 1-Brom-6,7–isopropylidendioxy-heptan

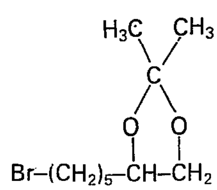

Zu 41 g 1-Brom-6,7-dihydroxyheptan und 22,2 g 2,2-Dimethoxypropan in 100 ml Aceton werden unter Rühren bei Raumtemperatur unter Stickstoff 0,1 ml konz. Schwefelsäure hinzugefügt. Nach 4 Stunden werden 0,6 g Natriumcarbonat zugegeben. Nach einstündigem Weiterrühren wird abfiltriert, bei vermindertem Druck eingeengt und der Rückstand einer fraktionierten Vakuumdestillation unterworfen.
Ausbeute: 38 g (77,9% der Theorie)
Siedepunkt (0,4 m bar) 73–76°C
Brechungsindex $n_D^{20}$ = 1,4656

d) 1,3-Diethyl-7-(6,7-dihydroxyheptyl)-xanthin

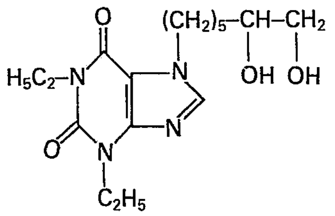

13,9 g 1,3-Diethylxanthin, 18 g 1-Brom-6,7-isopropylidendioxy-heptan und 9,5 g Kaliumcarbonat werden in 100 ml Dimethylformamid 7 Stunden unter Rühren auf 100°C erwärmt. Nach Einengen bei vermindertem Druck versetzt man den Rückstand mit verdünnter Natronlauge und extrahiert mehrmals mit Methylenchlorid. Die gesammelten Methylenchloridphasen werden mit Wasser gewaschen, getrocknet und eingeengt. Der Rückstand wird bei 100–115°C Badtemperatur und 0,01 m bar in einer Kugelrohrapparatur destilliert. Man erhält 17 g 1,3-Diethyl-7-(6,7-isopropylidendioxy-heptyl)-xanthin, die in 70 ml Schwefelsäure vom pH-Wert = 0,5 aufgenommen und 2 Stunden unter Rückfluss erhitzt werden. Nach dem Erkalten neutralisiert man, engt bei vermindertem Druck ein und kristallisiert den Rückstand aus Methylenchlorid/Diethylether um.
Ausbeute: 13,8 g (61,1% der Theorie)
Schmelzpunkt: 105°C
$C_{16}H_{26}N_4O_4$ (MG = 338,4)
Analyse: Berechnet: C 56,79% H 7,74% N 16,56%
Gefunden: C 56,83% H 7,70% N 16,67%

Das auf Stufe b) hergestellte 1-Brom-6,7-dihydroxyheptan kann auch ohne vorherige Umsetzung mit 2,2-Dimethoxypropan zum Dioxolan-Derivat unmittelbar zur Alkylierung des 1,3-Diethylxanthins in Stufe d) eingesetzt werden.

Beispiel 6
3,7-Diethyl-1-(4,5-dihydroxyhexyl)-xanthin

a) 1-Chlor-4-hexen

$Cl{-}(CH_2)_3{-}CH{=}CH{-}CH_3$

Zu einer Lösung von 330 g (3,3 Mol) 4-Hexen-1-ol (Organic Syntheses, Vol 55, Seite 62 ff) in 400 ml Pyridin gibt man unter Rühren und Eiskühlung aus einem Tropftrichter 476 g (4 Mol) Thionylchlorid so hinzu, dass die Reaktionstemperatur 55°C nicht übersteigt. Anschliessend wird 1 Stunde lang auf 80°C erwärmt. Danach lässt man den Ansatz langsam abkühlen, versetzt mit Wasser und extrahiert mehrmals mit Diethylether. Die vereinigten Etherextrakte werden mit gesättigter Natriumhydrogencarbonat-Lösung ausgeschüttelt, mit Wasser neutral gewaschen, über Natriumsulfat getrocknet und unter Normaldruck eingedampft. Durch fraktionierte Destillation des Rückstandes unter vermindertem Druck erhält man gaschromatographisch reines 1-Chlor-4-hexen.
Ausbeute: 196,2 g (50,1% der Theorie)
Siedepunkt (140 m bar) 80°C
Berechnungsindex $n_D4^{21}$ = 1,4400

b) 3,7-Diethyl-1-(4,5-dihydroxyhexyl)-xanthin

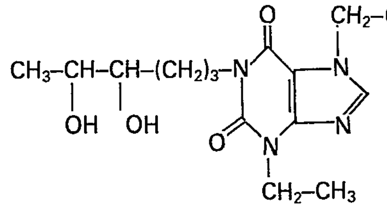

20,8 g (0,1 Mol) 3,7-Diethylxanthin, 15,2 g (0,11 Mol) Kaliumcarbonat und 13,0 g (0,11 Mol) 1-Chlor-4-hexen werden in 600 ml Dimethylformamid 18 Stunden lang bei 110°C gerührt. Nach dem Abkühlen wird filtriert, unter vermindertem Druck eingeengt und der Rückstand in Chloroform aufgenommen. Durch Ausschütteln mit 1N Natronlauge entfernt man das nicht umgesetzte 3,7-Diethylxanthin, wäscht die organische Phase mit Wasser neutral, trocknet über Natriumsulfat und destilliert das Lösungsmittel im Umlaufverdampfer ab. Nach dem Trocknen des festen Abdampfrückstandes erhält man 27,5 g (94,7% der Theorie) rohes 3,7-Diethyl-1-(4-hexenyl)-xanthin,

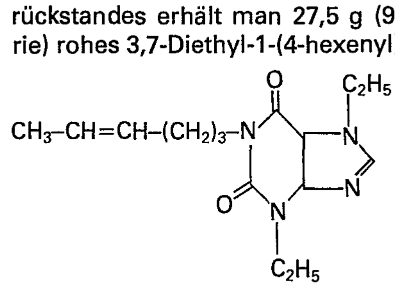

das nach Lösen in 350 ml Chloroform und Zugabe von 23,1 g (0,114 Mol) 3-Chlorperbenzoesäure (85%ig) 48 Stunden bei Raumtemperatur und unter Stickstoffatmosphäre gerührt wird. Danach schüttelt man zunächst mit 10%iger Natriumdithionitlösung bis zum Verschwinden der Jod-Stärke-Reaktion und dann mit 10%iger Natriumhydrogencarbonat-Lösung aus, wäscht mit Wasser neutral und salzfrei, trocknet und engt unter vermindertem Druck ein. Es werden 29,0 g (100% der Theorie) rohes, 3,7-Diethyl-1-(4,5-epoxyhexyl)-xanthin

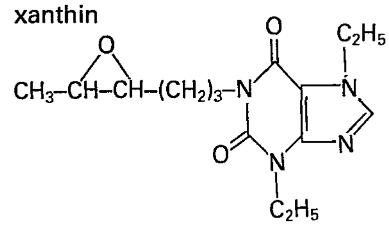

erhalten, die man nach Aufnahme in ein Lösungsmittelgemisch aus 150 ml Tetrahydrofuran und

100 ml Wasser und Versetzen mit 0,46 ml Perchlorsäure (70%ig) 90 Stunden bei Raumtemperatur rührt. Anschliessend wird mit gesättigter Natriumhydrogencarbonat-Lösung neutralisiert, das Lösungsmittel unter vermindertem Druck abdestilliert und der Rückstand an Kieselgel mit Chloroform als Laufmittel chromatographiert. Hierbei erhält man 26,4g (86% der Theorie) eines dünnschichtchromatographisch fast reinen, kristallinen Produktes, das aus Essigester/Petroläther umkristallisiert wird.

Ausbeute: 21,5g (70% der Theorie)
Schmelzpunkt: 91–93°C
$C_{15}H_{24}N_4O_3$ (MG = 324,4)
Analyse: Berechnet: C 55,54% H 7,46% N 17,27%
Gefunden: C 55,54% H 7,59% N 16,97%

Dieselbe Verbindung wird durch einstufige Oxidation von 3,7-Diethyl-1-(4-hexenyl)-xanthin mit Osmiumtetroxid analog Beispiel 2 C erhalten.

Beispiel 7
3-Butyl-1-(4,5-dihydroxypentyl)-7-methylxanthin

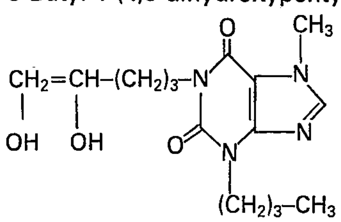

Man erhitzt 33,3g (0,15 Mol) 3-Butyl-7-methylxanthin, 24,3g (0,16 Mol) 1-Brom-4-penten und 22,1g (0,16 Mol) Kaliumcarbonat in 500 ml Dimethylformamid 15 Stunden lang unter Rühren auf 100°C. Nach dem Abkühlen des Reaktionsgemisches wird unter vermindertem Druck eingeengt, der Rückstand in Methylenchlorid aufgenommen, filtriert, das Filtrat mit 1 N Natronlauge ausgeschüttelt, die organische Phase mit Wasser neutral gewaschen, über Natriumsulfat getrocknet und im Umlaufverdampfer vom Lösungsmittel befreit. Dabei werden 42,2g (97% der Theorie) rohes, kristallines 3-Butyl-7-methyl-1-(4-pentenyl)-xanthin

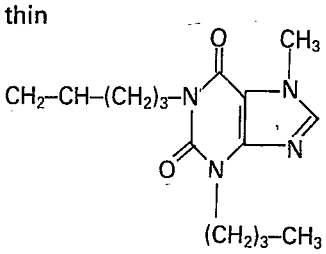

erhalten, die man in 550 ml Chloroform löst, mit 35,6g (0,175 Mol) 3-Chlorperbenzoesäure (85%ig) versetzt und nach Überlagerung mit Stickstoff 67 Stunden lang bei Raumtemperatur rührt.

Ausschütteln mit 10%iger Natriumdithionitlösung bis zum Verschwinden der Jod-Stärke-Reaktion, Waschen zunächst mit 10%iger Natriumhydrogencarbonat-Lösung und dann mit Wasser, Trocknen über Natriumsulfat und Einengen unter vermindertem Druck liefert 37,7g (84,7% der Theorie) rohes 3-Butyl-1-(4,5-epoxypentyl)-7-methylxanthin,

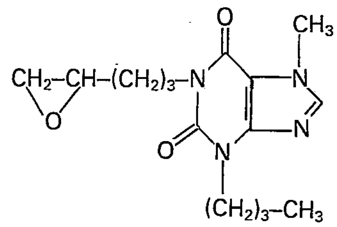

das ohne Zwischenreinigung der hydrolytischen Oxiranringöffnung unterworfen wird. Hierzu löst man die 37,7g Epoxid in einem Gemisch aus 200 ml Tetrahydrofuran und 135 ml Wasser und tropft unter Rühren bei Raumtemperatur 0,61 ml Perchlorsäure (70%ig) innerhalb von etwa 10 Minuten hinzu. Nach 14-stündigem Rühren bei Raumtemperatur wird mit gesättigter Natriumhydrogencarbonat-Lösung neutralisiert und unter vermindertem Druck eingedampft. Das zurückbleibende ölige Rohprodukt (100% der Theorie) lässt sich durch Säulenchromatographie in Kieselgel mit Choroform/Methanol (Volumenverhältnis 10/1) als Fliessmittel und anschliessende Umkristallisation aus Essigester unter Zusatz von Petrolether in der Siedehitze bis zur Trübung reinigen.

Ausbeute: 29,2g (73,2% der Theorie)
Schmelzpunkt: 76–78°C
$C_{15}H_{24}N_4O_4$ (MG = 324,4)
Analyse: Berechnet: C 55,54% H 7,46% N 17,27%
Gefunden: C 55,38% H 7,45% N 17,62%

Zu derselben Verbindung gelangt man durch direkte Dihydroxylierung der C=C-Doppelbindung von 3-Butyl-7-methyl-1-(4-pentenyl)-xanthin mit Osmiumtetroxid analog Beispiel 2 C).

Beispiel 8
1,7-Diethyl-3-(5,6-dihydroxyhexyl)-xanthin

a) 1,7-Diethylxanthin

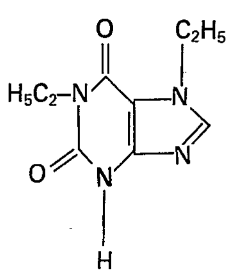

18g 3-Benzyl-1,7-diethylxanthin (F. 119°C) werden in 1500 ml Eisessig in Gegenwart von 2,5g 10%-Palladium auf Aktivkohle bei 80°C und 3,4 bar während 47 Stunden unter Schütteln hydriert. Nach dem Abkühlen überlagert man mit Stickstoff, filtriert den Katalysator ab und engt unter vermindertem Druck ein. Der Rückstand wird mit einem Gemisch aus 250 ml Methylenchlorid und 100 ml 1 n Natronlauge gelöst. Nach Nachwaschen der Methylenchlroridphase mit 1 n Natronlauge werden die vereinigten wässrigen Phasen unter Rühren durch Zutropfen von 33%iger Schwefelsäure auf pH = 6 gestellt. Nach Neutralwaschen und Trocknen des entstandenen Niederschlages erhält man 8,1g (64,5% der Theorie) 1,7-Diethylxanthin vom Schmelzpunkt 204–205°C.

Aus der Methylenchloridphase werden 4,6g 3-Benzyl-1,7-diethylxanthin zurückgewonnen.

b) 1,7-Diethyl-3-(5,6-dihydroxyhexyl)-xanthin

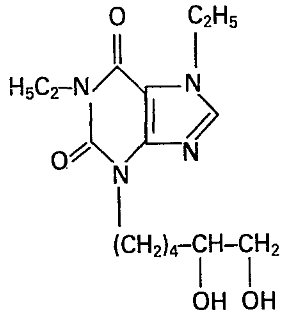

Das Gemisch aus 7g 1,7-Diethylxanthin, 7,2g 1-Chlor-5,6-isopropylidendioxy-hexan, 5g Kaliumcarbonat und 50 ml Dimethylformamid wird 8 Stunden bei 120°C gerührt. Nach Einengen bei vermindertem Druck nimmt man mit 50 ml 1 n Natronlauge auf und extrahiert mit Methylenchlorid. Die Methylenchloridphase wird mit verdünnter Natronlauge nachgewaschen, neutralgewaschen, getrocknet und bei vermindertem Druck eingeengt. Den Rückstand destilliert man bei 0,01 bis 0,02 m bar und 120–150°C Badtemperatur in einem Dünnschichtverdampfer und erhält 11,5g 1,7-Diethyl-3-(5,6-isopropylidendioxy-hexyl)-xanthin. Dieses wird mit 325 ml Methanol und 80 ml Wasser aufgenommen und nach Zugabe von 0,4 ml Perchlorsäure (70%ig) 1 Stunde bei 70°C gerührt. Nach dem Abkühlen auf Zimmertemperatur neutralisiert man mit Natriumcarbonatlösung und engt bei vermindertem Druck zur Trockne ein. Es wird mit 200 ml Methylenchlorid aufgenommen und der Extrakt eingeengt.

Den Rückstand reinigt man durch Säulenchromatographie an Kieselgel mit Methylenchlorid/Ethanol (Volumenverhältnis 8/2) als Laufmittel und durch anschliessende Umkristallisation aus Methylenchlorid/Diethylether.

Ausbeute: 7,9g (72,5% der Theorie)
Schmelzpunkt: 115–116°C
$C_{15}H_{24}N_4O_4$ (MG = 324,4)
Analyse: Berechnet: C 55,54% H 7,46% N 17,27%
Gefunden: C 55,37% H 7,51% N 17,08%

Zu diesem Diol gelangt man auch durch Umsetzung von 1,7-Diethyl-3-(5-hexenyl)-xanthin mit Osmiumtetroxid analog Beispiel 2 C oder durch Epoxidation von vorgenannter Xanthinverbindung gefolgt von der sauren Hydrolyse des Epoxidringes analog den Beispielen 3, 4 und 7.

Beispiel 9
7-(2,3-Dihydroxybutyl)-1,3-dipropylxanthin

a) 1-Chlor-2,3-epoxy-butan

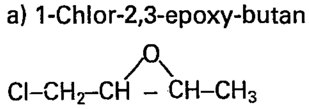

Zu einer Lösung von 244g 3-Chlor-perbenzoesäure (85%ig) in 1,5 l Chloroform werden bei Eiskühlung innerhalb einer Stunde unter Rühren 93,4g Crotylchlorid (97%ig) zugetropft. Nach 70 Stunden Weiterrühren bei Raumtemperatur saugt man vom Niederschlag ab, wäscht das Filtrat mit 10%iger Natriumdithionitlösung (bis der Jod-Stärke-Test negativ ist) mit gesättigter Natriumbicarbonatlösung und mit Wasser. Nach Tocknen über Natriumsulfat wird über eine Füllkörperkolonne fraktioniert destilliert.

Ausbeute: 58g (54,3% der Theorie)
Siedepunkt (133 m bar) 70–73°C
Brechungsindex $n_D^{20}$ = 1,4327
$C_4H_7C10$ (MG = 106,55)
Analyse: Berechnet: C 45,09% H 6,62% Cl 33,27%
Gefunden: C 45,28% H 6,78% Cl 33,30%

b) 1-Chlor-2,3-dihydroxybutan

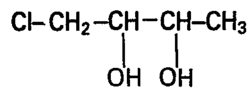

57g 1-Chlor-2,3-epoxybutan werden im Gemisch mit 500 ml Wasser, 800 ml Tetrahydrofuran und 1,2 ml Perchlorsäure (70%ig) 7 Tage bei Raumtemperatur gerührt. Nach Neutralisation mit Natriumbicarbonatlösung engt man unter vermindertem Druck zur Trockne ein und nimmt den Rückstand mit 2 l Diethylether auf. Die etherische Lösung wird getrocknet und bei vermindertem Druck eingeengt.

Ausbeute: 54g (81% der Theorie)

c) 1-Chlor-2,3-isopropylidenidoxybutan

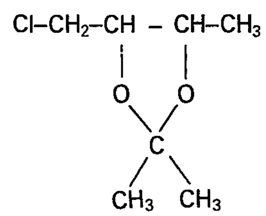

Zu einer Lösung von 53g 1-Chlor-2,3-dihydroxybutan in 50 ml Aceton und 71g 2,2-dimethoxypropan wird in einer Stickstoffatmosphäre unter Rühren 1 ml Perchlorsäure (70%ig) innerhalb 6 Minuten bei Raumtemperatur eingetropft. Nach einer weiteren Stunde Rühren bei Raumtemperatur rührt man eine halbe Stunde mit 5 g feingepulvertem Natriumbicarbonat, filtriert und destilliert das Filtrat über eine Füllkörperkolonne.

Ausbeute: 36,8g (52,5% der Theorie)
Siedepunkt (6,5 m bar) 49–53°C

d) 7-(2,3-Dihydroxybutyl)-1,3-dipropylxanthin

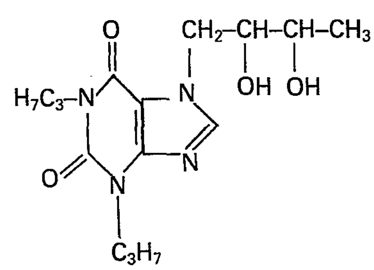

Das Gemisch aus 47,2g 1,3-Dipropylxanthin, 33,6g 1-Chlor-2,3-isopropyliden-dioxybutan,

28,2 g Kaliumcarbonat und 300 ml Dimethylformamid wird 8 Stunden bei 120°C gerührt. Man engt bei vermindertem Druck ein und nimmt den Rückstand mit 250 ml 1 n Natronlauge und 500 ml Methylenchlorid auf. Nach Nachwaschen der wässrigen Phase mit Methylenchlorid werden die vereinigten Methylenchloridphasen mit 1 n Natronlauge und Wasser gewaschen, getrocknet und bei vermindertem Druck eingeengt. Den Rückstand destilliert man bei 0,03–0,07 m bar und 90°C Badtemperatur im Kugelrohr. Man erhält 26,8 g 7-(2,3-Isopropylidendioxybutyl)-1,3-dipropylxanthin (91,5% der Theorie bezogen auf umgesetztes 1,3-Dipropyl-xanthin), das

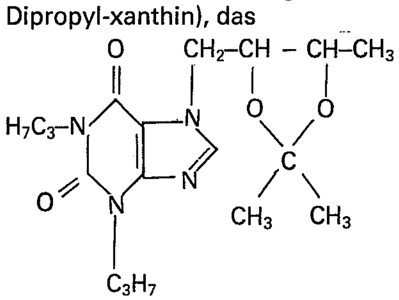

im Gemisch mit 700 ml Tetrahydrofuran, 100 ml Wasser und 0,8 ml Perchlorsäure (70%ig) 1 Stunde bei 70°C gerührt wird. Nach Neutralisation mit Natriumbicarbonatlösung engt man bei vermindertem Druck ein, nimmt den Rückstand mit 800 ml Methylenchlorid auf, trocknet und engt unter vermindertem Druck ein. Der Rückstand wird durch Umkristallisation aus Methylenchlorid/Diethylether gereinigt.

Ausbeute: 20,9 g (87,6% der Theorie)
Schmelzpunkt: 99–101°C
$C_{15}H_{24}N_4O_4$ (MG = 324,39)
Analyse: Berechnet: C 55,54% H 7,46% N 17,27%
Gefunden: C 55,49% H 7,49% N 17,18%

Die vorgenannten und die auf analoge Weise hergestellten Verbindungen sind in Tabelle 1 zusammengefasst.

Tabelle 1
Verbindung gemäss Formel I

| Beispiel | $R^1$ | $R^2$ | $R^3$ | Schmelzpunkt °C | hergestellt analog Beispiel Nr. |
|---|---|---|---|---|---|
| 1 | $-C_3H_7$ | $-C_2H_5$ | $-(CH_2)_4-CH-CH_2OH$<br>OH | 96–98 | siehe Beschreibung |
| 2 | $-C_2H_5$ | $-C_2H_5$ | $-(CH_2)_4-CH-CH_2OH$<br>OH | 94–95 | siehe Beschreibung |
| 3 | $-CH_3$ | $-CH_3$ | $-(CH_2)_4-CH-CH_2OH$<br>OH | 98–100 | siehe Beschreibung |
| 4 | $-C_4H_9$ | $-C_4H_9$ | $-(CH_2)_2-CH-CH_2OH$<br>OH | 92–93 | siehe Beschreibung |
| 5 | $-C_2H_5$ | $-C_2H_5$ | $-(CH_2)_5-CH-CH_2OH$<br>OH | 105 | siehe Beschreibung |
| 6 | $-(CH_2)_3-CH-CH-CH_3$<br>OH OH | $-C_2H_5$ | $-C_2H_5$ | 91–93 | siehe Beschreibung |
| 7 | $-(CH_2)_3-CH-CH_2OH$<br>OH | $-C_4H_9$ | $-CH_3$ | 76–78 | siehe Beschreibung |
| 8 | $-C_2H_5$ | $-(CH_2)_4-CH-CH_2OH$<br>OH | $-C_2H_5$ | 115–116 | siehe Beschreibung |
| 9 | $-C_3H_7$ | $-C_3H_7$ | $-(CH_2)-CH-CH-CH_3$<br>OH OH | 99–101 | siehe Beschreibung |

Tabelle 1 (Fortsetzung)

| Beispiel | $R^1$ | $R^2$ | $R^3$ | Schmelzpunkt °C bzw. $n_D^{20}$ | hergestellt analog Beispiel Nr. |
|---|---|---|---|---|---|
| 10 | $-CH_2)_4-CH-CH_2OH$<br>OH | $-C_3H_7$ | $-CH_3$ | 112–113 | 2 A) |
| 11 | $-C_{10}H_{21}$ | $-CH_3$ | OH<br>$-(CH_2)_2-CH-CH_2OH$ | 107–108 | 3, 4 |
| 12 | $-C_3H_7$ | $-C_3H_7$ | OH<br>$-(CH_2)_2-CH-CH_2OH$ | 93–95 | 3, 4 |
| 13 | $-C_3H_7$ | $-C_3H_7$ | OH<br>$-(CH_2)_3-CH-CH_2OH$ | 83–84 | 3,4 |
| 14 | $-C_2H_5$ | $-C_4H_9$ | OH<br>$-(CH_2)_3-CH-CH_2OH$ | 79–80 | 3,4 |
| 15 | $-C_3H_7$ | $-C_2H_5$ | $-(CH_2)_3-CH-CH_2OH$<br>OH | 82–84 | 3,4 |
| 16 | $-C_2H_5$ | $-C_2H_5$ | $-(CH_2)_2-CH-CH_2OH$<br>OH | 132–134 | 3,4 |
| 17 | $-C_2H_5$ | $-CH_3$ | $-(CH_2)_4-CH-CH_2OH$<br>OH | 111–113 | 1 |
| 18 | $-C_3H_7$ | $-CH_3$ | $-(CH_2)_4-CH-CH_2OH$<br>OH | 78–79 | 1 |
| 19 | $-C_4H_9$ | $-CH_3$ | $-(CH_2)_4-CH-CH_2OH$<br>OH | 81–83 | 1 |
| 20 | 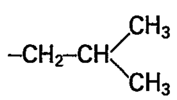 | $-CH_3$ | $-(CH_2)_4-CH-CH_2OH$<br>OH | 62–64 | 1 |
| 21 | $-C_5H_{11}$ | $-CH_3$ | $-(CH_2)_4-CH-CH_2OH$<br>OH | 84–85 | 1 |
| 22 | 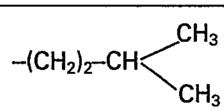 | $-CH_3$ | $-(CH_2)_4-CH-CH_2OH$<br>OH | 71–73 | 1 |
| 23 | 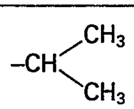 | $-CH_3$ | $-(CH_2)_4-CH-CH_2OH$<br>OH | 83–85 | 1 |
| 24 | $-CH_3$ | $-C_2H_5$ | $-(CH_2)_4-CH-CH_2OH$<br>OH | 100–101 | 1 |
| 25 | 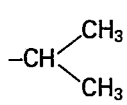 | $-C_2H_5$ | $-(CH_2)_4-CH-CH_2OH$<br>OH | $n_D^{20} = 1,5443$ | 1 |

Tabelle 1 (Fortsetzung)

| Beispiel | $R^1$ | $R^2$ | $R^3$ | Schmelzpunkt °C | hergestellt analog Beispiel Nr. |
|---|---|---|---|---|---|
| 26 | $-C_4H_9$ | $-C_2H_5$ | $-(CH_2)_4-CH-CH_2OH$<br>\|<br>OH | 78–82 | 1 |
| 27 | $CH_3$<br>$-CH_2-CH<$<br>$CH_3$ | $-C_2H_5$ | $-(CH_2)_4-CH-CH_2OH$<br>\|<br>OH | 80–81 | 1 |
| 28 | $-C_5H_{11}$ | $-C_2H_5$ | $-(CH_2)_4-CH-CH_2OH$<br>\|<br>OH | 77–78 | 1 |
| 29 | $CH_3$<br>$-(CH_2)_2-CH<$<br>$CH_3$ | $-C_2H_5$ | $-(CH_2)_4-CH-CH_2OH$<br>\|<br>OH | 79 | 1 |
| 30 | $-CH_3$ | $-C_3H_7$ | $-(CH_2)_4-CH-CH_2OH$<br>\|<br>OH | 82–84 | 1 |
| 31 | $-C_2H_5$ | $-C_3H_7$ | $-(CH_2)_4-CH-CH_2OH$<br>\|<br>OH | 80–82 | 1 |
| 32 | $-C_3H_7$ | $-C_3H_7$ | $-(CH_2)_4-CH-CH_2OH$<br>\|<br>OH | 78–80 | 2 A) |
| 33 | $-C_3H_7$ | $-C_2H_5$ | OH OH<br>\| \|<br>$-(CH_2)_3-CH-CH-CH_3$ | 91–92 | 6, 1 |
| 34 | $-CH_3$ | $-C_3H_7$ | OH OH<br>\| \|<br>$-(CH_2)_3-CH-CH-CH_3$ | 149 | 6, 1 |
| 35 | $-CH_3$ | $-(CH_2)_4-CH-CH_2OH$<br>\|<br>OH | $-CH_3$ | 153–154 | 8 |
| 36 | $-(CH_2)_2-CH-CH_2OH$<br>\|<br>OH | $-CH_3$ | $-C_4H_9$ | 88–89 | 7 |
| 37 | $-(CH_2)_2-CH-CH_2OH$<br>\|<br>OH | $-CH_3$ | $-C_6H_{13}$ | 43–45 | 7 |
| 38 | $-(CH_2)_2-CH-CH_2OH$<br>\|<br>OH | $-C_2H_5$ | $-C_2H_5$ | 94–96 | 7 |
| 39 | $-(CH_2)_2-CH-CH_2OH$<br>\|<br>OH | $-CH_3$ | $-C_{12}H_{25}$ | 63–65 | 7 |
| 40 | $-(CH_2)_3-CH-CH_2OH$<br>\|<br>OH | $-CH_3$ | $-C_3H_7$ | 83–85 | 7 |
| 41 | $-(CH_2)_3-CH-CH_2OH$<br>\|<br>OH | $-CH_3$ | $-C_5H_{11}$ | 105–107 | 7 |

Tabelle 1 (Fortsetzung)

| Beispiel | $R^1$ | $R^2$ | $R^3$ | Schmelzpunkt °C | hergestellt analog Beispiel Nr. |
|---|---|---|---|---|---|
| 42 | $-(CH_2)_3-CH-CH_2OH$<br>\|<br>OH | $-C_2H_5$ | $-C_2H_5$ | 114–116 | 7 |
| 43 | $-(CH_2)_4-CH-CH_2OH$<br>\|<br>OH | $-CH_3$ | $-C_3H_7$ | 97–100 | 2 A) |
| 44 | $-(CH_2)_4-CH-CH_2OH$<br>\|<br>OH | $-CH_3$ | $-C_4H_9$ | 80–81 | 2 A) |
| 45 | $-(CH_2)_4-CH-CH_2OH$<br>\|<br>OH | $-CH_3$ | $-C_5H_{11}$ | 87–88 | 2 A) |
| 46 | $-(CH_2)_4-CH-CH_2OH$<br>\|<br>OH | $-CH_3$ | $-C_6H_{13}$ | 54–56 | 2 A) |
| 47 | $-(CH_2)_4-CH-CH_2OH$<br>\|<br>OH | $-C_2H_5$ | $-C_2H_5$ | 92–93 | 2 A) |
| 48 | $-(CH_2)_4-CH-CH_2OH$<br>\|<br>OH | $-C_2H_5$ | $-C_3H_7$ | 107–108 | 2 A) |
| 49 | $-(CH_2)_4-CH-CH_2OH$<br>\|<br>OH | $-C_2H_5$ | $-C_4H_9$ | 82–83 | 2 A) |
| 50 | OH OH<br>\| \|<br>$-(CH_2)_3-CH-CH-CH_3$ | $-CH_3$ | $-C_3H_7$ | 107 | 6 |
| 51 | $-C_3H_7$ | $-C_2H_5$ | $-(CH_2)_5-CH-CH_2OH$<br>\|<br>OH | 102–103 | 1, 5 |

Tabelle 2
Zwischenprodukte mit einem Strukturelement der Formel IV
(Anordnung der Reste $R^1$, $R^2$ und $R^3$ wie bei Formel I)

| Verb. | $R^1$ | $R^2$ | $R^3$ | Fp°C |
|---|---|---|---|---|
| 1a | $-(CH_2)_3-CH-CH_2$<br>\ /<br>O | $C_2H_5$ | $CH_3$ | 76–77 |
| 2a | $-(CH_2)_3-CH-CH_2$<br>\ /<br>O | $C_4H_9$ | $CH_3$ | (Beisp. 7) |
| 3a | $-(CH_2)_3-CH-CH-CH_3$<br>\ /<br>O | $C_2H_5$ | $C_2H_5$ | (Beisp. 6b) |
| 4a | $-(CH_2)_4-CH-CH_2$<br>\ /<br>O | $CH_3$ | $CH_3$ | 99–100 |

Tabelle 2 (Fortsetzung)

| Verb. | $R^1$ | $R^2$ | $R^3$ | Fp°C |
|---|---|---|---|---|
| 5a | 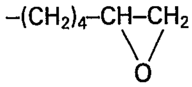 | $C_2H_5$ | $CH_3$ | 80 |
| 6a | $CH_3$ | $CH_3$ | $-(CH_2)_4-CH-CH_2$<br>O | 59–60 |
| 7a | $C_2H_5$ | $C_2H_5$ | $-(CH_2)_3-CH-CH_2$<br>O | 58–59 |
| 8a | $C_2H_5$ | $C_2H_5$ | $-(CH_2)_4-CH-CH_2$<br>O | 58–59 |
| 9a | $C_4H_9$ | $C_4H_9$ | $-(CH_2)_2-CH-CH_2$<br>O | 52–53 |

Tabelle 3

Zwischenprodukte mit einem Strukturelement der Formel IX ($R^6 = CH_3$; $R^4$, $R^7$ und n: siehe Tabelle; Anordnung von $R^1$, $R^2$ und $R^3$ wie bei Formel I)

| Verb. | $R^1$ | $R^2$ | $R^3$ | Kugelrohrdest. bei °C | mbar | Fp°C |
|---|---|---|---|---|---|---|
| 1b | H | $CH_3$ | n=4, $R^4$=H<br>$R^7=CH_3$ | | | 187–188 |
| 2b | H | $C_2H_5$ | n=4, $R^4=R^7$<br>=H | | | 146 |
| 3b | H | $C_2H_5$ | n=4, $R^4$=H<br>$R^7=CH_3$ | | | 123–124 |
| 4b | $CH_3$ | $C_2H_5$ | n=4, $R^4$=H<br>$R^7=CH_3$ | 150 | 0,02 | |
| 5b | $C_2H_5$ | $C_2H_5$ | n=4, $R^4$=H<br>$R^7=CH_3$ | 130–150 | 0,027* | |
| 6b | $C_2H_5$ | $C_2H_5$ | n=5, $R^4$=H,<br>$R^7$=Ch$_3$ | 100–115 | 0,01 | |
| 7b | $C_2H_5$ | $C_3H_7$ | n=4, $R^4$=H,<br>$R^7=CH_3$ | 150–160 | 0,02 | |
| 8b | $C_3H_7$ | $C_2H_5$ | n=4, $R^4$=H,<br>$R^7=CH_3$ | 135–140 | 0,01 | |
| 9b | $C_3H_7$ | $C_2H_5$ | n=5, $R^4$=H,<br>$R^7=CH_3$ | 130 | 0,02 | |
| 10b | $C_3H_7$ | $C_3H_7$ | n=4, $R^4$=H<br>$R^7=CH_3$ | 140–150 | 0,05 | |
| 11b | $C_3H_7$ | $C_3H_7$ | n=1, $R^4$=H<br>$R^7=CH_3$ | 90 | 0,03–0,07 | |
| 12b | $C_4H_9$ | $C_2H_5$ | n=4, $R^4$=H<br>$R^7=CH_3$ | 120–130 | 0,03 | |

* Dünnschichtverdampfer

Tabelle 3 (Fortsetzung)

| Verb. | $R^1$ | $R^2$ | $R^3$ | Kugelrohrdest. bei °C | mbar | Fp°C |
|---|---|---|---|---|---|---|
| 13b | $C_5H_{11}$ | $CH_3$ | n=4, $R^4$=H $R^7=CH_3$ | 130–140 | 0,03 | |
| 14b | $C_5H_{11}$ | $C_2H_5$ | n=4, $R^4$=H $R^7=CH_3$ | 130–140 | 0,02 | |
| 15b | $C_2H_5$ | n=4, $R^4$=H $R^7=CH_3$ | $C_2H_5$ | 120–150 | 0,01–0,02* | |
| 16b | n=4, $R^4$=H, $R^7=CH_3$ | $C_2H_5$ | $C_3H_7$ | 140–150 | 0,2 | |
| 17b | n=4, $R^4$=H $R^7=CH_3$ | $C_3H_7$ | $CH_3$ | 140 | 0,2 | |

* Dünnschichtverdampfer

Beispie 52

Arzneimittelzubereitung: Für die Herstellung von 1000 Dragees werden 100g 3-Ethyl-7-(5,6-dihydroxyhexyl)-1-propylxanthin (Verbindung gemäss Beispiel 1), 20g Laktose, 30g Maisstärke, 8,5g Talkum, 0,5g kolloidale Kieselsäure und 1g Magnesiumstearat gemischt und zu Drageekernen von 160mg Gewicht verpresst, die anschliessend mit einem Dragiergemisch aus 40g Rohrzukker, 23,5g Talkum und sehr kleinen Zusätzen von Wachs, Titandioxid und arabischem Gummi so behandelt werden, dass das Endgewicht der Dragees jeweils 225 mg beträgt.

Beispiel 53

Arzneimittelzubereitung: Für die Herstellung von 1000 Dragees werden 111,8g 3-Ethyl-7-(5,6-isopropylidendioxyhexyl)-1-propylxanthin (aus Beispiel 1), 20g Laktose, 30g Maisstärke, 8,5g Talkum, 0,5g kolloidale Kieselsäure und 1g Magnesiumstearat gemischt und zu Drageekernen von 171,8mg Gewicht verpresst, die anschliessend mit einem Dragiergemisch aus 40g Rohrzucker, 23,5g Talkum und sehr kleinen Zusätzen von Wachs, Titanoxid und arabischem Gummi so behandelt werden, dass das Endgewicht der Dragees jeweils 240mg beträgt.

Beispiel 54

Arzneimittelzubereitung: Zur Herstellung von 1000 Dragees werden 100g 1,3-Diethyl-7-(5,6-epoxyhexy)-xanthin (aus Beispiel 2B), 20g Laktose, 30g Maisstärke, 8,5g Talkum, 0,5g kolloidale Kieselsäure und 1g Magnesiumstearat gemischt und zu Drageekernen von 160mg Gewicht verpresst, die anschliessend mit einem Dragiergemisch aus 40g Rohrzucker, 23,5g Talkum und sehr kleinen Zusätzen von Wachs, Titandioxid und arabischem Gummi so behandelt werden, dass das Endgewicht der Dragees jeweils 225mg beträgt.

Pharmakologische Prüfung und Ergebnisse

1. Bronchospasmolytische Wirkung

Die Prüfung der erfindungsgemässen Verbindungen auf bronchospasmolytische Aktivität erfolgte im wesentlichen mit der von H. Konzett und R. Rössler beschriebenen Versuchsanordnung (Arch. exp. Path. u. Pharmak. 195 (1940), 75 im Vergleich mit dem Standardtherapeutikum Theophyllin-Ethylendiamin sowie den beiden vorbeschriebenen 2,3-Dihydroxypropyl-Verbindungen Dyphyllin [7–(2,3-Dihydroxypropyl)-1,3-dimethylxanthin] und 7–(2,3-Dihydroxypropyl)-1,3-dipropylxanthin. Bei dieser Methode wird die Hemmung experimenteller Bronchialspasmen – ausgelöst durch intravenöse Gabe spasmogen wirkender Amine, wie Acetylcholin, Histamin und Serotonin – an Meerschweinchen beiderlei Geschlechts in Urethan-Narkose (1,25 g/kg i.p.) untersucht.

Die Prüfsubstanzen wurden in wässriger Lösung entweder intravenös (i.v.) oder intraduodenal (i.d.) verabreicht. Die $ED_{50}$-Werte, die jene Dosois in mg/kg darstellen, bei der der experimentell erzeugte Spasmus auf die Hälfte gegenüber jenem unbehandelter Tiere herabgesetzt ist, wurden graphisch aus den Dosiswirkungskurven bestimmt.

2. Akute Toxizität

Die Bestimmung der $LD_{50}$-Werte erfolgte standardgemäss über die innerhalb von 7 Tagen bei NMRI-Mäusen nach einmaliger intravenöser (i.v.), intraperitonealer (i.p.) bzw. oraler (p.o.) Gabe auftretende Mortalität.

Die Ergebnisse dieser Untersuchungen, die die Überlegenheit der erfindungsgemässen Verbindungen entsprechend Formel I gegenüber dem Standardpräparat Theophyllin-Ethylendiamin und den beiden anderen Vergleichssubstanzen demonstrieren (insbesondere auch unter Berücksichtigung des günstigeren Verhältnisses von $LD_{50}$ zu $ED_{50}$), sind in der nachfolgenden Tabelle 4 zusammengefasst.

Tabelle 4
Pharmakologische Testergebnisse

| Verbindung aus Beispiel | Applikationsart | Bronchospasmolytische Wirkung ($ED_{50}$ in mg/kg) gegenüber Acetylcholin | Histamin | Serotonin | Toxizität* $LD_{50}$ (Maus i. V., i. p. bzw. p. o.) mg/kg | | Therapeutischer Index $\frac{LD_{50}}{ED_{50}}$ |
|---|---|---|---|---|---|---|---|
| 1 | i. v. | 2,0 | 0,2 | 0,3 | i. v.: | 254 | 305 |
| | i. d. | 6,3 | 25,0 | 15,0 | p. o.: | 1450 | 94 |
| 2 | i. v. | 2,0 | 2,0 | 2,0 | i. v.: | 325 | 163 |
| 7 | i. v. | 3,0 | 6,5 | – | i. v.: | > 200 | > 42 |
| 8 | i. v. | 6,5 | 6,5 | 2,0 | i. v.: | > 300 | > 60 |
| 10 | i. v. | 3,0 | 3,0 | 3,0 | i. v.: | 250 | 83 |
| 12 | i. v. | 6,5 | 10,0 | 10,0 | i. p.: | > 600 | > 68 |
| 13 | i. v. | 6,5 | – | – | i. p.: | 900 | 138 |
| 17 | i. v. | 2,0 | 6,5 | 6,5 | i. v.: | > 300 | > 60 |
| 18 | i. v. | 6,5 | 6,5 | 6,5 | i. v.: | 300 | 46 |
| 19 | i. v. | 2,0 | 2,0 | 3,0 | i. v.: | 147 | 63 |
| 21 | i. v. | 3,0 | 3,0 | 3,0 | i. v.: | 155 | 52 |
| 22 | i. v. | 1,0 | 3,0 | 6,5 | i. v.: | 167 | 48 |
| 24 | i. v. | 6,5 | 2,0 | 10,0 | i. v.: | 300 | 49 |
| 25 | i. v. | 2,0 | 6,5 | 2,0 | i. v.: | > 400 | >114 |
| 26 | i. v. | 2,0 | 1,6 | 0,6 | i. v.: | 205 | 146 |
| 28 | i. v. | 2,0 | 3,0 | 3,0 | i. v.: | 200 | 75 |
| 30 | i. v. | 6,5 | 2,0 | – | i. v.: | 250 | 59 |
| 33 | i. v. | 6,0 | 3,0 | 2,0 | i. v.: | > 200 | > 55 |
| 51 | i. v. | 2,0 | 2,0 | – | i. v.: | 300 | 150 |
| Theophylin- | i. v. | 8,3 | 5,6 | 7,3 | i. v.: | 195 | 28 |
| Ethylen-diamin | i. d. | 40,0 | 50,0 | 50,0 | p. o.: | 550 | 12 |
| Dyphyllin | i. v. | >30,0 | > 30,0 | > 30,0 | i. v.: | 886**) | < 30 |
| 7-(2,3-Di-=hydroxy-=propyl)-1,3-dipropylxanthin | i. v. | >10,0 | 6,5 | 10.0 | i. v.: | 287**) | < 32 |

*) bestimmt nach Litchfield und Wilcoxon (J. Pharmacol. exp. Ther. 96, (1949,) 99)
*) bestimmt nach Litchfield und Wilcoxon (J. Pharmacol. exp. Ther. 96 (1949), 99)
**) gemäss DOS 27 16 402

Die eindeutige Überlegenheit der erfindungsgemässen Verbindungen insbesondere gegenüber dem am häufigsten zur Therapie obstruktiver Atemwegserkrankungen eingesetzten Xanthinderivat, dem Theophyllin-Ethylendiamin (Aminophyllin), liess sich auch in weiteren Spezialversuchen eindrucksvoll bestätigen:

Da es als erwiesen gilt, dass neben den in Tabelle 4 aufgeführten biogenen Aminen Acetylcholin, Histamin und Serotonin auch das Bradykinin eine wichtige Rolle als Mediatorsubstanz bei der Auslösung von Asthmaanfällen spielt, wurde die Hemmwirkung auf den am Meerschweinchen mit Bradykinin ausgelösten Bronchospasmus untersucht. Hierbei erwies sich beispielsweise die Verbindung aus Beispiel 1 bei intravenöser (i. v.) Verabreichung mit einer $ED_{50}$ von 1–3 mg/kg etwa 3 mal und nach intraduodenaler (i. d.) Applikation mit einer $ED_{50}$ von 4,0–6,3 mg/kg ca. 6 mal stärker wirksam als Theophyllin-Ethylendiamin, für das die entsprechenden $ED_{50}$-Werte zu 3–10 mg/kg i. v. und 25–40 mg/kg i. d. ermittelt wurden.

Auf den am präsensibilisierten Meerschweinchen mit Ovalbumin (1 mg/kg i. v.) induzierten Bronchospasmus, der mit den herkömmlichen Xanthinderivaten kaum zu beeinflussen ist, üben die Verbindungen der Formel I ebenfalls eine starke Hemmwirkung aus. So liegt beispielsweise der $ED_{50}$-Wert für die Verbindung des Beispiels 1 zwischen 6 und 12 mg/kg i. v., während das Theophyllin-Ethylendiamin mit Dosen bis zu 10 mg/kg keine Wirkung in dieser Versuchsanordnung zeigt.

Die überlegene bronchospasmolytische Aktivität der Xanthine gemäss Formel I konnte schliesslich auch im Lungenfunktionstest am narkotisierten Hund anhand der Hemmung der mit Acetylcholin-, Histamin- und Ascarisextrakt-Aerosolen ausgelösten bronchospastischen Reaktionen nachgewiesen werden.

So zeigte etwa die Verbindung des Beispiels 1 bereits mit 12 mg/kg i. v. eine signifikante Hemmwirkung, während sich Theophyllin in Dosen bis zu 20 mg/kg i. v. als wirkungslos erwies.

Wie bereits eingangs erwähnt, steht der klinisch gut etablierten bronchospasmolytischen Wirkung des Theophyllin-Grundkörpers der erhebliche Nachteil einer sehr engen therapeutischen Breite verbunden mit gravierenden Nebenwirkungen, vor allem im kardiovaskulären Bereich (hypotensive Aktivität, Abnahme der cerebralen Durchblutung) und im zentralen Nervensystem (z.B. Unruhe, Schlaflosigkeit, Schwindel) gegenüber. Besonders störend wird von Patienten und Klinikern die zentrale Erregung empfunden, da sie häufig zu Schlaflosigkeit führt und damit den Allgemeinzustand des Asthmatikers nachhaltig beeinträchtigt. Als Ausdruck dieser zentralen Erregung erhöht Theophyllin-Ethylendiamin die Spontanmotilität weisser männlicher Mäuse nach oraler Verabreichung von 30 mg/kg um 186% über einen Zeitraum von 7 Stunden. Laut DE-A-27 16 402 rufen auch die beiden anderen Vergleichspräparate Dyphyllin und 7-(2,3-Dihydroxypropyl)-1,3-dipropylxanthin eine zentrale Stimulierung bei Mäusen hervor; wenngleich diese Effekte deutlich schwächer als beim Theophyllin ausgeprägt sind. Demgegenüber besitzen die erfindungsgemässen Verbindungen der Formel (I) keine zentralerregende Wirkungskomponente, sondern üben im Gegenteil eine leicht dämpfende Wirkung auf das zentrale Nervensystem aus, die aus therapeutischer Sicht besonders vorteilhaft zu beurteilen ist. So wird die Spontanmotilität der Mäuse beispielsweise nach oraler Gabe von 50 mg/kg der Verbindung des Beispiels 1 um 53% für die Dauer von 11 Stunden herabgesetzt.

Die an Ratten und Hunden im Vergleich mit Theophyllin-Ethylendiamin durchgeführten Kreislaufuntersuchungen haben gezeigt, dass die Verbindungen der Formel (I), wenn überhaupt, deutlich geringere hypotensive Aktivität besitzen und keine Abnahme der cerebralen Durchblutung verursachen.

**Patentansprüche für die Vertragsstaaten: BE, CH, DE, FR, GB, IT, LI, LU, NL, SE**

1. Arzneimittel, dadurch gekennzeichnet, dass sie Verbindungen der Formel (I) enthalten,

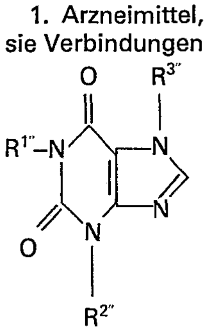
I

worin einer der Reste $R^1$, $R^2$ oder $R^3$ eine Dihydroxyalkylgruppe der Formel $CH_2OH\text{-}CHOH\text{-}(CH_2)_n\text{-}$ oder $CH_3\text{-}CHOH\text{-}CHOH\text{-}(CH_2)_{n-1}\text{–}$ mit n= 2-6 bedeutet und die beiden anderen Reste geradkettige oder verzweigte Alkylgruppen mit bis zu 12 C-Atomen in der Position von $R^1$ und $R^3$ und bis zu 4 C-Atomen in der Position von $R^2$ darstellen, wobei die Summe der C-Atome dieser beiden Alkylsubstituenten höchstens 14 beträgt.

2. Arzneimittel nach Anspruch 1, dadurch gekennzeichnet, dass sie die Verbindungen der Formel (I) in Prodrug-Form enthalten, und zwar in Form von Epoxyden bzw. Acetalen, aus der erst im Organismus die therapeutisch wirksamen Dihydroxyalkylxanthine mit den im Anspruch 1 definierten Substituenten $R^1$, $R^2$ und $R^3$ durch Biotransformation freigesetzt werden.

3. Verbindungen der Formel (I) gemäss Anspruch 1 oder 2 mit der Massgabe, dass $R^2$ und $R^3$ nicht gleichzeitig Methyl sind, wenn $R^1$ einen 4,5- oder 5,6-Dihydroxyhexylrest darstellt.

4. Ausführungsform nach Anspruch 1 oder 3, dadurch gekennzeichnet, dass in den Verbindungen der Formel (I) $R^1$ oder $R^2$ einen $CH_2OH\text{-}CHOH\text{-}(CH_2)_n$-Rest mit n= 3 oder 4 darstellt und die beiden Alkylsubstituenten $R^2$ und $R^3$ bzw. $R^1$ und $R^3$ zusammen 3 bis 6 C-Atome umfassen.

5. Ausführungsform nach Anspruch 1 oder 3, dadurch gekennzeichnet, dass in den Verbindungen der Formel (I) $R^3$ eine $CH_2OH\text{-}CHOH\text{-}(CH_2)_n$-Gruppe mit n= 2 bis 5 oder eine 4,5-Dihydroxyhexylgruppe bedeutet und die Summe der C-Atome der beiden Alkylreste $R^1$ und $R^2$ 3 bis 7 beträgt.

6. Ausführungsform nach Anspruch 1 oder 3, dadurch gekennzeichnet, dass die Verbindungen der Formel (I) 1,3-Dialkyl-7-(5,6-dihydroxyhexyl)-xanthine sind, deren Alkylreste $R^1$ und $R^2$ zusammen 3 bis 7 C-Atome beinhalten, vorzugsweise aber das 3-Äthyl-7-(5,6-dihydroxyhexyl)-1-propylxanthin ist.

7. Verfahren zur Herstellung von Xanthinderivaten der Formel (I) gemäss einem oder mehreren der Ansprüche 3 bis 6, dadurch gekennzeichnet, dass man eine Verbindung der Formel (II)

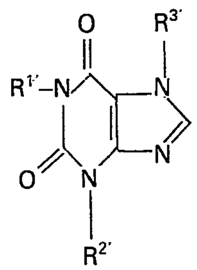
II

in der einer der Reste $R^{1'}$, $R^{2'}$ oder $R^{3'}$ eine Alkenylgruppe der Formel (IIIa) oder (IIIb) ist

$-(CH_2)_n\text{-}CH{=}CH_2$ III a

$-(CH_2)_{n-1}\text{-}CH{=}CH\text{-}CH_3$ IIIb

wobei n jeweils 2-6 ist und die beiden anderen Substituenten Wasserstoff oder wie bei Formel (I) gemäss einem oder mehreren der Ansprüche 3 bis 6 definiertes Alkyl darstellen,

a) mit geeigneten Oxidationsmitteln an der olefinischen Doppelbindung zu einem Epoxyalkylxanthin mit dem Strukturelement der Formel (IVa) oder (IVb)

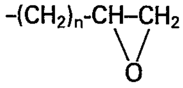
IVa

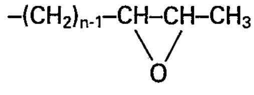
IVb

umsetzt und dessen Oxiranring unter Bildung eines Dihydroxyalkylxanthins mit dem Strukturmerkmal der Formel (Va) bzw. (Vb)

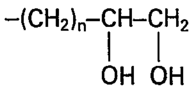 Va

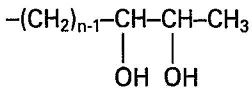 Vb

hydrolytisch öffnet oder

b) mit üblichen Oxidationsmitteln an der olefinischen Doppelbindung direkt zu dem Dihydroxyalkylxanthin mit dem durch Formel (Va) bzw. (Vb) gekennzeichneten Strukturelement dihydroxyliert und anschliessend jene der nach a) oder b) erhaltenen Diole, die noch Wasserstoff in den Positionen von $R^{1'}$, $R^{2'}$ und/oder $R^{3'}$ tragen, gegebenenfalls in Gegenwart basischer Mittel oder in Form ihrer Salze, mit Alkylierungsmitteln der Formel $R^4$-X (VI), in der X Halogen oder eine Sulfonsäureester- oder Phosphorsäureester-Gruppierung und $R^4$ die bei Formel (I) gemäss einem oder mehreren der Ansprüche 3 bis 6 definierten Alkylreste bedeuten, zu den Verbindungen der Formel (I) alkyliert, oder dass man eine Verbindung der Formel (VII)

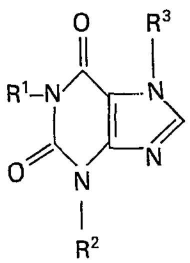 VII

in der höchstens zwei der Substituenten $R^{1''}$ bis $R^{3''}$ für entsprechend einem oder mehreren der Ansprüche 3 bis 6 definiertes Alkyl stehen und höchstens zwei dieser Reste Wasserstoff bedeuten, gegebenenfalls in Gegenwart basischer Mittel oder in Form ihrer Salze,

c) mit Alkylierungsmitteln der Formel (VIIIa) bzw. (VIIIb)

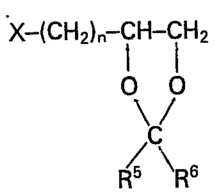 VIIIa

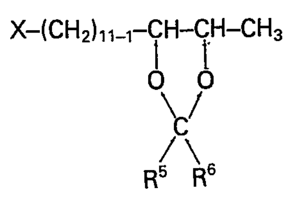 VIIIb

mit jeweils n= 2–6, wobei $R^5$ und $R^6$ unabhängig voneinander Wasserstoff, niederes Alkyl, Phenylalkyl mit bis zu 2 C-Atomen im Alkylteil oder, gegebenenfalls substituiertes, Phenyl und X Halogen oder eine Sulfonsäureester- oder Phosphorsäureester-Gruppierung bedeuten, zu einem di- oder trialkylierten Xanthin mit dem Strukturelement der Formel (IXa) oder (IXb)

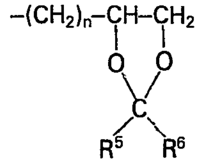 IXa

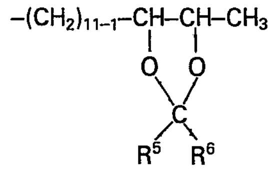 IXb

umsetzt und dessen 1,3-Dioxolanring unter Abspaltung von $R^5$–CO–$R^6$ und Bildung eines Dihydroxyalkylxanthins mit dem Strukturmerkmal der Formel (Va) bzw. (Vb)

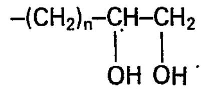 Va

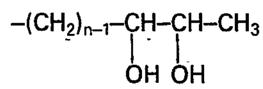 Vb

hydrolytisch öffnet oder

d) mit Alkylierungsmitteln der Formel (Xa) bwz. (Xb)

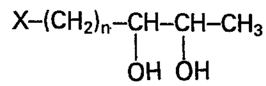 Xa

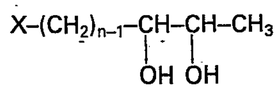 Xb

worin n=2–6 ist und X die oben angegebene Bedeutung hat, direkt zu dem Dihydroxyalkylxanthin mit dem durch Formel (Va) bzw. (Vb) gekennzeichneten Strukturelement umsetzt und anschliessend die nach c) oder d) erhaltenen Monoalkyldihydroxyalkylxanthine, die noch ein Wasserstoffatom in der Position von $R^{1''}$, $R^{2''}$ oder $R^{3''}$ tragen, gegebenenfalls in Gegenwart basischer Mittel oder in Form ihrer Salze, mit Alkylierungsmitteln der Formel $R^4$–X (VI), in der X und $R^4$ die bei Formel (VI) definierten Bedeutungen haben, zu den Verbindungen der Formel (I) umsetzt oder aber die nach c) hergestellten dialkylierten Xanthine mit dem Strukturelement der Formel (IXa) bzw. (IXb) zuerst mit den Verbindungen der Formel $R^4$–X (VI) alkyliert und dann den Dioxolanring unter Bildung der Dihydroxyalkylxanthine gemäss Formel (I) hydrolytisch spaltet.

8. Verbindungen der Formel (XI)

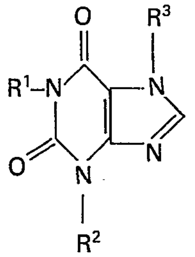

(XI)

in der einer der Reste $R^1$, $R^2$ und $R^3$ eine Gruppe der Formel (IVa) bzw. (IVb)

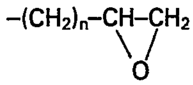

IVa

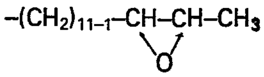

IVb

oder (IXa) bzw. (IXb)

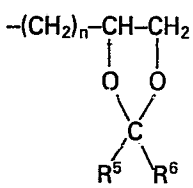

IXa

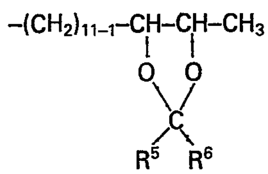

IXb

darstellt, wobei n=2–6 ist, $R^5$ und $R^6$ unabhängig voneinander Wasserstoff, Methyl, Phenylalkyl mit bis zu 2 C-Atomen im Alkylteil oder Phenyl sind, die übrigen der Reste $R^1$, $R^2$ und $R^3$ unabhängig voneinander Wasserstoff oder Alkyl mit 1 bis 12 C-Atomen in den Positionen $R^1$ und $R^3$ und mit 1 bis 4 C-Atomen in der Position $R^2$ bedeuten, wobei die Summe der Kohlenstoffatome dieser beiden Reste höchstens 14 beträgt.

9. Verbindungen nach Anspruch 8, worin $R^1$ oder $R^3$ der Rest der Formel (IVa) oder (IVb) ist und die anderen Reste unabhängig voneinander Alkyl mit 1 bis 4 C-Atomen sind.

10. Verbindungen nach Anspruch 8, worin $R^1$ Wasserstoff, Alkyl mit 1 bis 5 C-Atomen oder ein Rest der Formel (IXa) oder (IXb) ist und $R^2$ und $R^3$ unabhängig voneinander Alkyl mit 1 bis 3 C-Atomen oder ein Rest der Formel (IXa) oder (IXb) sind.

**Patentansprüche für den Vertragsstaat: AT**

1. Verfahren zur Herstellung von Xanthinderivaten der Formel (I)

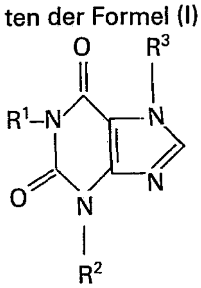

I

in der einer der Reste $R^1$, $R^2$ oder $R^3$ eine Dihydroxyalkylgruppe der Formel $CH_2OH{-}CHOH{-}(CH_2)_n{-}$ oder $CH_3{-}CHOH{-}CHOH{-}(CH_2)_{n-1}{-}$ mit n= 2–( bedeutet und die beiden anderen Reste geradkettige oder verzweigte Alkylgruppen mit bis zu 12 C-Atomen in der Position von $R^1$ und $R^3$ und bis zu 4 C-Atomen in der Position von $R^2$ darstellen, wobei die Summe der C-Atome dieser beiden Alkylsubstituenten jedoch höchstens 14 beträgt und wobei $R^2$ und $R^3$ nicht gleichzeitig Methyl sind, wenn $R^1$ einen 4,5- oder 5,6-Dihydroxyhexylrest darstellt, dadurch gekennzeichnet, dass man eine Verbindung der Formel (II),

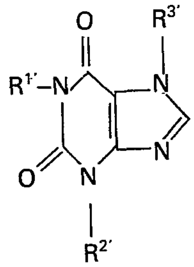

II,

in der einer der Reste $R^{1'}$, $R^{2'}$ oder $R^{3'}$ eine Alkenylgruppe der Formel (IIIa) oder (IIIb) ist

$-(CH_2)_n{-}CH{=}CH_2$ III a

$-(CH_2)_{n-1}{-}CH{=}CH{-}CH_3$ IIIb

wobei n jeweils 2–6 ist und die beiden anderen Substituenten Wasserstoff oder wie bei Formel (I) definiertes Alkyl darstellen,

a) mit geeigneten Oxidationsmitteln an der olefinischen Doppelbindung zu einem Epoxyalkylxanthin mit dem Strukturelement der Formel (IVa) oder (IVb)

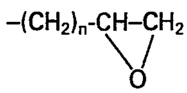

IVa

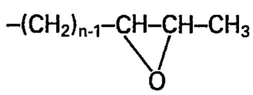

IVb

umsetzt und dessen Oxiranring unter Bildung eines Dihydroxyalkylxanthins mit dem Strukturmerkmal der Formel (Va) bzw. (Vb)

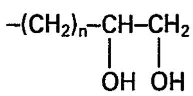

Va

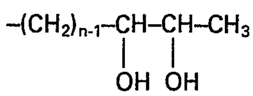

Vb

hydrolytisch öffnet oder

b) mit üblichen Oxidationsmitteln an der olefinischen Doppelbindung direkt zu dem Dihydroxyalkylxanthin mit dem durch Formel (Va) bzw. (Vb) gekennzeichneten Strukturelement dihydroxyliert und anschliessend jene der nach a) oder b) erhaltenen Diole, die noch Wasserstoff in den Positionen von $R^{1'}$, $R^{2'}$ und/oder $R^{3'}$ tragen, gegebenenfalls in Gegenwart basischer Mittel oder in Form ihrer Salze, mit Alkylierungsmitteln der Formel $R^4$-

X (VI), in der X Halogen oder eine Sulfonsäureester- oder Phosphorsäureester-Gruppierung und $R^4$ die bei Formel (I) definierten Alkylreste bedeuten, zu den Verbindungen der Formel (I) alkyliert, oder dass man eine Verbindung der Formel (VII)

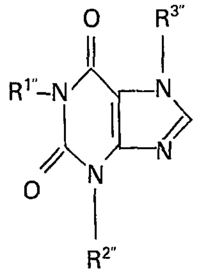

VII

in der höchstens zwei der Substituenten $R^{1''}$ bis $R^{3''}$ für bei Formel (I) definiertes Alkyl stehen und höchstens zwei dieser Reste Wasserstoff bedeuten, gegebenenfalls in Gegenwart basischer Mittel oder in Form ihrer Salze,

c) mit Alkylierungsmitteln der Formel (VIIIa) bzw. (VIIIb)

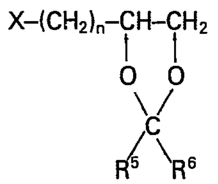

VIIIa

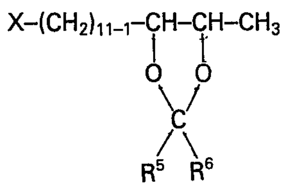

VIIIb

mit jeweils n= 2–6, wobei $R^5$ und $R^6$ unabhängig voneinander Wasserstoff, niederes Alkyl, Phenylalkyl mit bis zu 2 C-Atomen im Alkylteil oder, gegebenenfalls substituiertes, Phenyl und X Halogen oder eine Sulfonsäureester- oder Phosphorsäureester-Gruppierung bedeuten, zu einem di- oder trialkylierten Xanthin mit dem Strukturelement der Formel (IXa) oder (IXb)

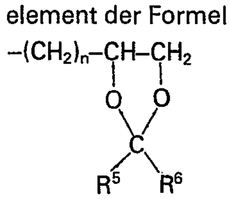

IXa

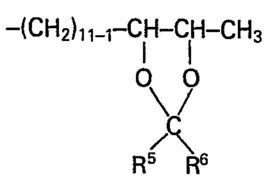

IXb

umsetzt und dessen 1,3-Dioxolanring unter Abspaltung von $R^5$–CO–$R^6$ und Bildung eines Dihydroxyalkylxanthins mit dem Strukturmerkmal der Formel (Va) bzw. (Vb)

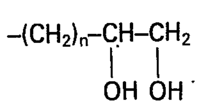

Va

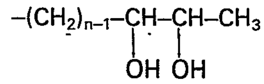

Vb

hydrolytisch öffnet oder

d) mit Alkylierungsmitteln der Formel (Xa) bwz. (Xb)

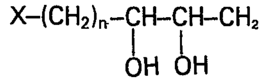

Xa

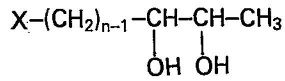

Xb

worin n=2–6 ist und X die oben angegebene Bedeutung hat, direkt zu dem Dihydroxyalkylxanthin mit dem durch Formel (Va) bzw. (Vb) gekennzeichneten Strukturelement umsetzt und anschliessend die nach c) oder d) erhaltenen Monoalkyldihydroxyalkylxanthine, die noch ein Wasserstoffatom in der Position von $R^{1''}$, $R^{2''}$ oder $R^{3''}$ tragen, gegebenenfalls in Gegenwart basischer Mittel oder in Form ihrer Salze, mit Alkylierungsmitteln der Formel $R^4$–X (VI), in der X und $R^4$ die bei Formel (VI) definierten Bedeutungen haben, zu den Verbindungen der Formel (I) umsetzt oder aber die nach c) hergestellten dialkylierten Xanthine mit dem Strukturelement der Formel (IXa) bzw. (IXb) zuerst mit den Verbindungen der Formel $R^4$–X (VI) alkyliert und dann den Dioxolanring unter Bildung der Dihydroxyalkylxanthine gemäss Formel (I) hydrolytisch spaltet.

2. Verfahren nach Anspruch 1, dadurch gekennzeichnet, dass man eine Verbindung der Formel (II), in der einer der Reste $R^{1'}$, $R^{2'}$ und $R^{3'}$ eine Alkenylgruppe der Formel (IIIa) bzw. (IIIb) ist, mit einer peroxidgruppenhaltigen Verbindung zum Oxiran oxydiert und dieses im wässrigen Medium bei 20 bis 100°C hydrolysiert.

3. Verfahren nach Anspruch 2, dadurch gekennzeichnet, dass die Oxydation mit Percarbonsäuren bei einer Temperatur von −10 bis +40°C durchgeführt wird.

4. Verfahren nach Anspruch 1, dadurch gekennzeichnet, dass man eine Verbindung der Formel (II), in der einer der Reste $R^{1'}$, $R^{2'}$ und $R^{3'}$ eine Alkenylgruppe der Formel (IIIa) bzw. (IIIb) ist, mit Wasserstoffperoxid, Chromchlorid, einem Permanganat, Jod, Selendioxid, Molybdän (VI) oxid oder Osmiumtetroxid oxidiert.

5. Verfahren nach Anspruch 4, dadurch gekennzeichnet, dass die Oxidation mit stöchiometrischen Mengen Osmiumtetroxid erfolgt und der intermediär gebildete Osmium(VI)-ester-Komplex reduktiv hydrolysiert wird.

6. Verfahren nach Anspruch 1, dadurch gekennzeichnet, dass man das bei der Ausführungsform c) erhaltene, den Dioxolanring enthaltende Xanthinderivat im wässrigen Medium bei Temperaturen von 20°C bis zum Siedepunkt des Reaktionsmediums hydrolytisch öffnet.

7. Verfahren nach einem oder mehreren der Ansprüche 1 bis 6, dadurch gekennzeichnet, dass in den Verbindungen der Formel (I) $R^1$ oder $R^2$ einen $CH_2OH–CHOH–(CH_2)_n$-Rest mit n = 3 oder 4

darstellt und die beiden Alkylsubstituenten $R^2$ und $R^3$ bzw. $R^1$ und $R^3$ zusammen 3 bis 6 C-Atome umfassen.

8. Verfahren nach einem oder mehreren der Ansprüche 1 bis 6, dadurch gekennzeichnet, dass in den Verbindungen der Formel (I) $R^3$ eine $CH_2OH{-}CHOH{-}(CH_2)_n$-Gruppe mit n = 2 bis 5 oder eine 4,5-Dihydroxyhexylgruppe bedeutet und die Summe der C-Atome der beiden Alkylreste $R^1$ und $R^2$ 3 bis 7 beträgt.

9. Verfahren nach Anspruch 8, dadurch gekennzeichnet, dass die Verbindungen der Formel (I) 1,3-Dialkyl-7-(5,6-dihydroxyhexyl-)xanthine sind, vorzugsweise aber das 3-Äthyl-7-(5,6-dihydroxyhexyl)-1-propylxanthin ist.

10. Verfahren zur Herstellung eines Arzneimittels, dadurch gekennzeichnet, dass man nach dem Verfahren eines oder mehrerer der Ansprüche 1 bis 9 eine Verbindung der Formel (I) herstellt, worin einer der Reste $R^1$, $R^2$ oder $R^3$ ein Dihydroxyalkylgruppe der Formel $CH_2OH{-}CHOH{-}(CH_2)_n$- oder $CH_3{-}CHOH{-}CHOH{-}(CH_2)_{n-1}{-}$ mit n = 2–6 bedeutet und die beiden anderen Reste geradkettige oder verzweigte Alkylgruppen mit bis zu 12 C-Atomen in der Position von $R^1$ und $R^3$ und bis zu 4 C-Atomen in der Position von $R^2$ darstellen, wobei die Summe der C-Atome dieser beiden Alkylsubstituenten jedoch höchstens 14 beträgt, und diese mit einem therapeutisch annehmbaren Hilfsmittel konfektioniert.

**Claims for the Contracting States BE, CH, DE, FR, GB, IT, LI, LU, NL, SE**

1. Medicaments characterized in that they contain compounds of the formula (I)

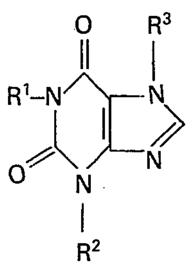

I

wherein one of the radicals $R^1$, $R^2$ or $R^3$ denotes a dihydroxyalkyl group of the fromula $CH_2OH$-$CHOH$-$(CH_2)_n$- or $CH_3$-$CHOH$-$CHOH$-$(CH_2)_{n-1}$-with n = 2–6 and the two other radicals represent straight-chain or branched alkyl groups having in positions $R^1$ and $R^3$ up to 12 carbon atoms and in position $R^2$ up to 4 carbon atoms, the total of carbon atoms in these two alkyl substituents being at most 14.

2. Medicaments according to claim 1, characterized in that they contain the compounds of formula (I) in prodrugform, i.e. in the form of epoxides or acetals, from which prodrug from the therapeutically active dihydroxyalkyl xanthines with the substituents $R^1$, $R^2$ and $R^3$ defined in claim 1 are liberated only in the organism by biotransformation.

3. Compounds of the formula (I) according to claim 1 or 2 with the proviso, that $R^2$ and $R^3$ are not simultaneously methyl, if $R^1$ is a 4,5- or 5,6-dihydroxyhexyl group.

4. Embodiment according to claims 1 to 6, characterized in that in the compounds of formula (I) $R^1$ or $R^2$ represents a $CH_2OH$-$CHOH$-$(CH_2)_n$- radical eith n = 3 or 4 and the two alkyl substituents $R^2$ and $R^3$ or $R^1$ and $R^3$ together habe 3 to 6 carbon atoms.

5. Embodiment according to claim 1 to 3, characterized in that in the compounds of formula (I) $R^3$ is a $CH_2$-$OH$-$CHOH$-$(CH_2)_n$ group with n = 2–5 or a 4,5-dihydroxyhexyl group and the sum of the carbon atoms of both alkyl groups $R^1$ and $R^2$ is from 3 to 7.

6. Embodiment according to claim 1 or 3, characterized in that the compounds of formula (I) are 1,3-dialkyl-7-(5,6-dihydroxyhexyl)-xanthines the alkyl groups $R^1$ and $R^2$ together comprising 3 to 7 carbon atoms, but wherein the compound of formula (I) preferably is the 3-ethyl-7-(5,6-dihydroxyhexyl)-1-propylxanthine.

7. A process for ghe preparation of xanthine derivatives of the formula (I) according ot one or more of claims 3 to 6, cahracterized in that a compound of the formula

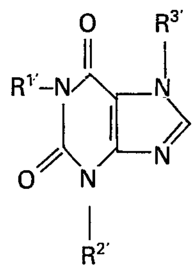

II

in which one of the radicals $R^{1'}$, $R^{2'}$ or $R^{3'}$ is an alkenyl group of the formula (IIIa) or (IIIb)

$-(CH_2)_n-CH=CH_2$ IIIa

$-(CH_2)_{n-1}-CH=CH-CH_3$ IIIb

n in both cases being 2–6, and the other two substituents representing hydrogen or alkyl as defined in formula (I) according to one or more of the claims 3 to 6,

a) is reacted on the olefinic double bond with a suitable oxidizing agent to give an epoxyalkylxanthine having the structural element of the formula (IVa) or (IVb)

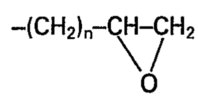

IVa

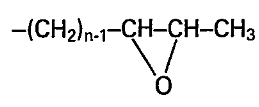

IVb

and its oxirane ring is hydrolytically opened with formation of a dihydroxyalkylxanthine having the structural unit of the formula (Va) or (Vb)

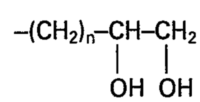

Va

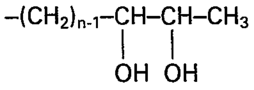

Vb

or

b) is dihydroxylated with a customary oxidizing agent on the olefinic double bond to give directly the dihydroxyalkylxanthine having the structural element characterized by formula (Va) or (Vb) respectively, and then those diols obtained according to a) or b) which still carry hydrogen in the positions of $R^{1'}$, $R^{2'}$ and/or $R^{3'}$, are alkylated, optionally in the presence of basic agents or in the form of their salts, with alkylating agents of the formula

$R^4$–X VI

in which X denotes halogen or a sulfinc acid ester or phosphoric acid ester grouping and $R^4$ denotes the alkyl radicals defined in relation to formula (I) in one or more of claims 3 to 6, to give the compounds of the formula (I), or that a compound of the formula (VII)

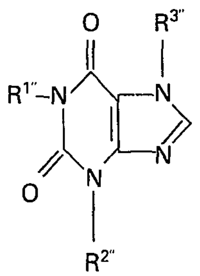

VII

in which at most two of the substituents $R^{1''}$ to $R^{3''}$ represent the alkyl defined in one or more of the claims 3 to 6 and a maximum of thwo of these radicals denote hydrogen, is reacted, optionally in the presence of basic agents or in the form of their salts,

c) with alkylating agents of the formula (VIIIa) or (VIIIb)

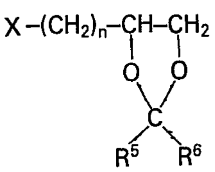

VIIIa

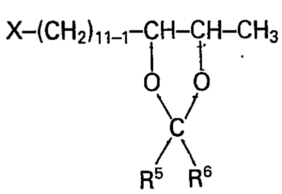

VIIIb

with n = 2–6 in each of which, $R^5$ and $R^6$, independently of one another, denoting hydrogen, lower alkyl, phenylalkyl having up to 2 carbon atoms in the moiety or, optionally substituted, phenyl and X denoting halogen or a sulfonic acid ester or phophoric acid ester grouping, to give a dialkylated or trialkylated xanthine having the structural element of the formula (IXa) or (IXb)

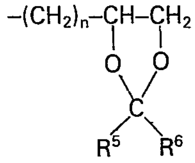

IXa

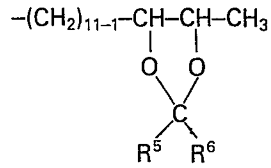

IXb

and its 1,3-dioxolane ring is hydrolytically openend, while splitting off $R^5$-CO-$R^6$ and forming a dihydroxyalkylxanthine having the structural unit of the formula (Va) or (Vb)

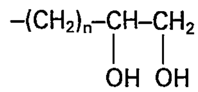

Va

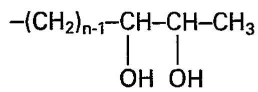

Vb

or

d) with an alkylating agent of the formula (Xa) or (Xb)

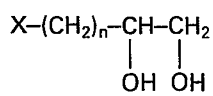

Xa

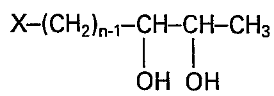

Xb

in which n = 2–6 and X has the aforementioned meaning, directly to give the dihydroxyalkylxanthine having the structural element characterized by the formula (Va) or (Vb), and the monoalkyldihydroxyalkylxanthines obtained according to c) or d) which still carry a hydrogen atom in the position of $R^{1''}$, $R^{2''}$ or $R^{3''}$, are then reacted, optionally in the presence of basic agents in the form of their salts, with aklylating agents of the fomula $R^4$–X (VI) in which X and $R^4$ have the meanings defined for formula (VI), to give the compounds of the formula I, or the dialkylated xanthines prepared according to c) having the structural element of the formula (IXa) or (IXb) are first alkylated with the compound of the formula $R^4$–X (VI) and the dioxolane ring is then hydrolytically cleaved with formation of the dihydroxyalkylxanthine according to formula I.

8. Compounds of the formula (XI)

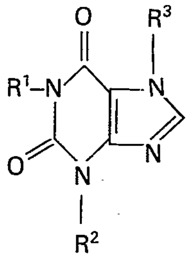

XI

in which one of the radicals $R^1$, $R^2$ and $R^3$ represents a group of the formula (IVa) or (IVb)

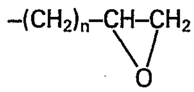

IVa

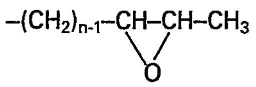

IVb

or (IXa) or (IXb)

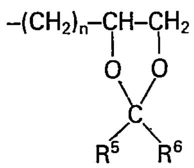

IXa

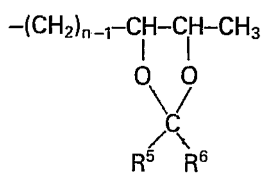

IXb

wherein n is 2–6, $R^5$ and $R^6$, independently of one another, are hydrogen, methyl, phenylalkyl having up to 2 C atoms in the alkyl moiety or phenyl, the remaining of the radicals $R^1$, $R^2$ and $R^3$, independently of one another, denote hydrogen or alkyl having 1 to 12 C atoms in the positions $R^1$ and $R^3$ and having 1 to 4 C atoms in the position $R^2$, the total of carbon atoms in these two radicals being a maximum of 14.

9. Compounds according to claim 8, wherein $R^1$ or $R^3$ is the radical of the formula (IVa) or (IVb) and the other radicals, independently of one another, are alkyl having 1 to 4 C atoms.

10. Compounds according to claim 8, wherein $R^1$ is hydrogen, alkyl having 1 to 5 C atoms or a radical of the formula (IXa) or (IXb) and $R^2$ and $R^3$, independently of one another, are alkyl having 1 to 3 C atoms or a radical of the formula (IXa) or (IXb).

**Claims for the Contracting State AT**

1. A process for the manufacture of xanthine derivatives of the formula (I)

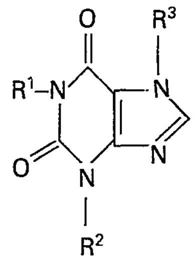

I

wherein one of the radicals $R^1$, $R^2$ or $R^3$ denotes a dihydroxylalkyl group of the formula $CH_2OH-CHOH-(CH_2)_n-$ or $CH_3-CHOH-CHOH-(CH_2)_{n-1}-$ with n = 2–6 and the two other radicals represent straight-chain or branched alkyl groups having in positions $R^1$ and $R^3$ up to 12 carbon atoms and in position $R^2$ up to 4 carbon atoms, the total of carbon atoms in these two alkyl substituents being, however, at most 14 and wherein $R^2$ and $R^3$ are not simultaneously methyl, if $R^1$ is a 4,5- or 5,6-dihydroxyhexyl radical, characterized in that a compound of the formula

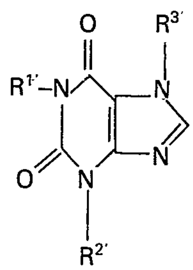

II

in which one of the radicals $R^{1'}$, $R^{2'}$ or $R^{3'}$ is an alkenyl group of the formula (IIIa) or (IIIb)

$-(CH_2)_n-CH=CH_2$ IIIa

$-(CH_2)_{n-1}-CH=CH-CH_3$ IIIb

n in both cases being 2–6, and the other two substituents representing hydrogen or alkyl as defined in formula (I)

a) is reacted on the olefinic double bond with a suitable oxidizing agent to give an epoxyalkylxanthine having the structural element of the formula (IVa) or (IVb)

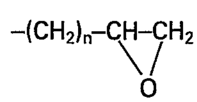

IVa

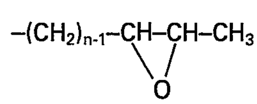

IVb

and its oxirane ring is hydrolytically opened with formation of a dihydroxyalkylxanthine having the structural unit of the formula (Va) or (Vb)

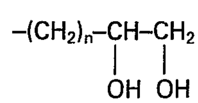

Va

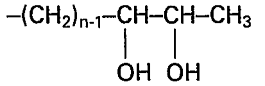
Vb

or

b) is dihydroxylated with a customary oxidizing agent on the olefinic double bond to give directly the dihydroxyalkylxanthine having the structural element characterized by formula (Va) or (Vb) respectively, and then those diols obtained according to a) or b) which still carry hydrogen in the positions of $R^{1'}$, $R^{2'}$ and/or $R^{3'}$, are alkylated, optionally in the presence of basic agents or in the form of their salts, with alkylating agents of the formula

$R^4$–X VI

in which X denotes halogen or a sulfonic acid ester or phosphoric acid ester grouping and $R^4$ denotes the alkyl radicals defined in relation fo formula (I) to give the compounds of the formula (I), or that a compound of the formula (VII)

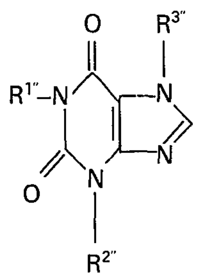
VII

in which at most two of the substituents $R^{1''}$ to $R^{3''}$ represent the alkyl defined in in relation to formula (I) and a maximum of thwo of these radicals denote hydrogen, is reacted, optionally in the presence of basic agents or in the form of their salts,

c) with alkylating agents of the formula (VIIIa) or (VIIIb)

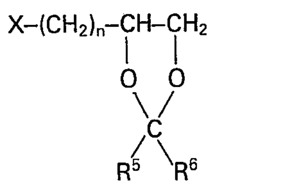
VIIIa

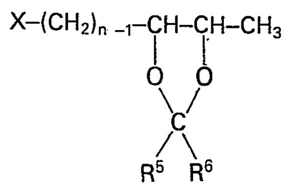
VIIIb

with n = 2–6 in each of which, $R^5$ and $R^6$, independently of one another, denoting hydrogen, lower alkyl, phenylalkyl having up to 2 carbon atoms in the moiety or, optionally substituted, phenyl und X denoting halogen or a sulfonic acid ester or phophoric acid ester grouping, to give a dialkylated or triakylated xanthine having the structural element of the formula (IXa) or (IXb)

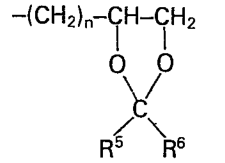
IXa

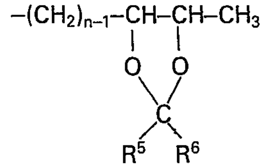
IXb

and its 1,3-dioxolane ring is hydrolytically openend, while splitting off $R^5$-CO-$R^6$ and forming a dihydroxyalkylxanthine having the structural unit of the formula (Va) or (Vb)

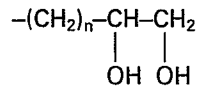
Va

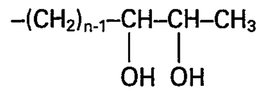
Vb

or

d) with an alkylating agent of the formula (Xa) or (Xb)

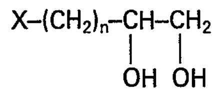
Xa

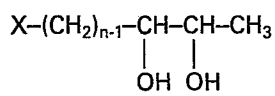
Xb

in which n = 2–6 and X has the aforementioned meaning, directly to give the dihydroxyalkylxanthine having the structural element characterized by the formula (Va) or (Vb), and the monoalkyldihydroxyalkylxanthines obtained according to c) or d) which still carry a hydrogen atom in the position of $R^{1''}$, $R^{2''}$ or $R^{3''}$, are then reacted, optionally in the presence of basic agents in the form of their salts, with alkylating agents of the formula $R^4$–X (VI) in which X and $R^4$ have the meanings defined for formula (VI), to give the compounds of the formula I, or the dialkylated xanthines prepared according to c) having the structural element of the formula (IXa) or (IXb) are first alkylated with the compounds of the formula $R^4$–X (VI) and the dioxolane ring is then hydrolytically cleaved with formation of the dihydroxyalkylxanthines according to formula I.

2. Process according to claim 1, characterized in that a compound of the formula (II) in which one of the radicals $R^{1'}$ and $R^{2'}$ and $R^{3'}$ is an alkenyl group of the formula (IIIa) or (IIIb) is oxidized with a compound containing a peroxy group to give an oxirane and this is hydrolyzed in an aqueous medium at 20 to 100°C.

3. Process according to claim 2, characterized in that the oxidation is conducted with peroxycarboxylic acids at a temperature in the range from −10 to +40°C.

4. Process according to claim 1, characterized in that a compound of the formula (II) in which one of the radicals $R^{1'}$ and $R^{2'}$ and $R^{3'}$ is an alkenyl group of the formula (IIIa) or (IIIb) is oxidized with hydrogen peroxide, chromyl chloride, a permanganate, iodine, selenium dioxide, molybdenum (VI) oxide or osmium tetroxide.

5. Process according to claim 4, characterized in that the oxidation is conducted with stoeichiometric amounts of osmium tetroxide and the osmium (VI)-ester-complex which is intermediately formed is hydrolyzed under reduction.

6. Process according to claim 1, characterized in that the xanthine derivative obtained according to embodiment c) and containing the dioxolane ring is hydrolytically opened in an aqueous medium at a temperature in the range from 20°C to the boiling point of the reaction medium.

7. Process according to one or more of claims 1 to 6, characterized in that in the compounds of formula (I) $R^1$ oder $R^2$ represents a $CH_2OH-CHOH-(CH_2)_n-$radical with n = 3 or 4 and the two alkyl substituents $R^2$ and $R^3$ or $R^1$ and $R^3$ together have 3 to 6 carbon atoms.

8. Process according to one or more of claims 1 to 6, characterized in that in the compounds of formula (I) $R^3$ is a $CH_2OH-CHOH-(CH_2)_n$ group with n = 2–5 or a 4,5-dihydroxyhexyl group and the sum of the carbon atoms of both alkyl groups $R^1$ and $R^2$ is from 3 to 7.

9. Process according to claim 8, characterized in that the compounds of formula (I) are 1,3-dialkyl-7-(5,6-dihydroxyhexyl)-xanthines, but preferably is the 3-ethyl-7-(5,6-dihydroxyhexyl)-1-propylxanthine.

10. Process for the manufacture of a medicament, characterized in that a compound of formula (I) is produced according to the process of one or more of claims 1 to 9, in which one of the radicals $R^1$, $R^2$ or $R^3$ is a dihydroxyalkyl group of the formula $CH_2OH-CHOH-(CH_2)_n-$ or $CH_3-CHOH-CHOH-(CH_2)_{n-1}-$ with n = 2–6 and the other two radicals represent straight-chain or branched alkyl groups having up to 12 carbon atoms in the position of $R^1$ and $R^3$ and up to 4 carbon atoms in the position of $R^2$, the total of carbon atoms of these two alkyl substituents being, however, at most 14, and that this compound of formula (I) is then processed with a therapeutically acceptable auxiliary agent.

**Revendications pour les Etats contractants BE CH DE FR GB IT LI LU NL SE**

1. Médicaments caractérisés par le fait qu'ils contiennent des composés de formule (I)

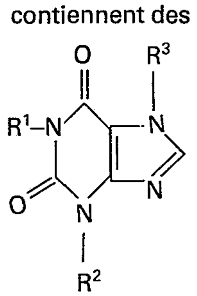

I

dans laquelle l'un des radicaux $R^1$, $R^2$ ou $R^3$ représente un groupe dihydroxy-alkyle de formule $CH_2OH-CHOH-(CH_2)_n-$ ou $CH_3-CHOH-CHOH-(CH_2)_{n-1}-$ où n = 2–6, et les deux autres radicaux représentent des groupes alkyle à chaîne droite ou ramifiée contenant jusqu'à 12 atomes de carbone à la position de $R^1$ et de $R^3$ et jusqu'à 4 atomes de carbone à la position de $R^2$, la somme des atomes de carbone de ces deux substituants alkyle étant égale à 14 au maximum.

2. Médicaments selon la revandication 1, caractérisés par le fait qu'ils contiennent les composés de formule (I) sous forme de précurseurs de médicaments, à savoir sous la forme d'époxydes ou d'acétals, à partir de laquelle les dihydroxyalkylxanthines à activité thérapeutique, contenant les substituants $R^1$, $R^2$ et $R^3$ définis dans la revendication 1, ne sont libérées qu'une fois dans l'organisme, par biotransformation.

3. Composés de formule (I) selon la revendication 1 ou 2, sous réserve que $R^2$ et $R^3$ ne sont pas simultanément des groupes méthyle lorsque $R^1$ représente un radical 4,5- ou 5,6-dihydroxyhexyle.

4. Mode de réalisation selon la revendication 1 ou 3, caractérisé par le fait que dans les composés de formule (I) $R^1$ our $R^2$ représente un groupe $CH_2OH-CHOH-(CH_2)_n$ où n = 3 ou 4 et les deux substituants alkyle $R^2$ et $R^3$ ou $R^1$ et $R^2$ comprennent ensemble de 3 à 6 atomes de carbone.

5. Mode de réalisation selon la revendication 1 ou 3, caractérisé par le fait que dans les composés de formule (I) $R^3$ représente un groupe $CH_2OH-CHOH-(CH_2)_n-$ où n = 2 à 5 ou un groupe 4,5-dihydroxy-hexyle, et que la somme des atomes de carbone des deux radicaux alkyle $R^1$ et $R^2$ va de 3 à 7.

6. Mode de réalisation selon la revendication 1 ou 3, caractérisé par le fait que les composés de formule (I) sont des 1,3-dialkyl-7-(5,6-dihydroxy-hexyl)-xanthines dont les radicaux alkyle $R^1$ et $R^2$ contiennent ensemble de 3 à 7 atomes de carbone, mais qui est préférablement la 3-éthyl-7-(5,6-dihydroxy-hexyl)-1-propylxanthine.

7. Procédé pour la préparation de dérivés de la xanthine de formule (I) selon une ou plusieurs des revendications 3 à 6, caractérisé par le fait que l'on transforme un composé de formule (II)

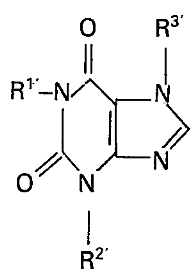

II

dans laquelle l'un des radicaux $R^{1'}$, $R^{2'}$ ou $R^{3'}$ est un groupe alcényle de formule (IIIa) ou (IIIb)

$-(CH_2)_n-CH=CH_2$ IIIa

$-(CH_2)_{n-1}-CH=CH-CH_3$ IIIb

dans lesquelles n est chaque fois égal à 2–6, et les deux autres substituants représentent chacun un atome d'hydrogène ou un groupe alkyle tel que défini pour la formule (I) selon une ou plusieurs des revendications 3 à 6,

a) au niveau de la double liaison éthylénique, avec des oxydants appropriés, en une époxy-alkylxanthine contenant l'élément structural de formule (IVa) ou (IVb)

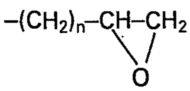
IVa

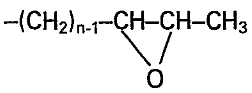
IVb

et que l'on ouvre par hydrolyse le cycle oxyrane de celui-ci avec formation d'une dihydroxy-alkylxanthine contenant l'élément stuctural caractéristique de formule (Va) ou (Vb)

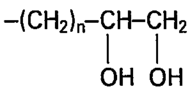
Va

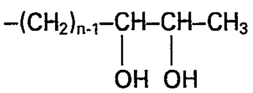
Vb

ou bien

b) qu'il est directement di-hydroxylé avec des oxydants usuels au niveau de la double liaison éthylénique en dihydroxy-alkylxanthine contenant l'élément stuctural caractérisé par la formule (Va) ou (Vb) et qu'ensuite ceux des diols obtenus suivant a) ou suivant b) qui comportent encore un atome d'hydrogène aux positions $R^{1'}$, $R^{2'}$ et/ou $R^{3'}$ sont alkylés en composés de formule (I), éventuellement en présence bases ou sous forme de leurs sels, avec des agents d'alkylation de formule

$R^4$–X (VI)

dans laquelle X représente un halogène ou un groupement ester sulfonique ou ester phosphorique et $R^4$ représente les radicaux alkyle définis pour la formule (I) selon une ou plusieurs des revendications 3 à 6, ou bien que l'on transforme un composé de formule (VII)

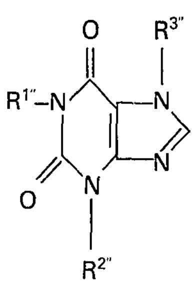

dans lequel deux au plus des substituants $R^{1''}$ à $R^{3''}$ représentent chacun un groupe alkyle défini selon une ou plusieurs des revendications 3 à 6 et deux au plus de ces radicaux représentent un atome d'hydrogène, éventuellement en présence de bases ou sous forme de ses sels,

c) avec des agents d'alkylation de formule (VIIIa) ou (VIIIb)

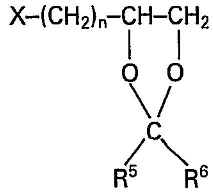
VIIIa

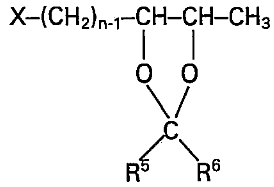
VIIIb

où n est chaque fois égal à 2–6, $R^5$ et $R^6$ représentant indépendamment l'un de l'autre un atome d'hydrogène, un groupe alkyle inférieur, phénylalkyle contenant jusqu'à 2 atomes de carbone dans le radical alkyle, ou un group phényle éventuellement substitué, et X représentant un halogène ou un groupement ester sulfonique ou ester phosphorique, en une xanthine di- ou tri-alkylée contenant l'élément structural de formule (IXa) ou (IXb)

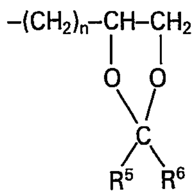
IXa

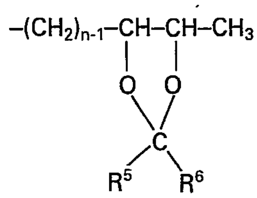
IXb

et que l'on ouvre par hydrolyse le cycle 1,3-dioxolanne de celui-ci avec séparation de $R^5$–CO–$R^6$ et formation d'une dihydroxy-alkylxanthine contenant l'élément structural caractéristique de formule (Va) ou (Vb)

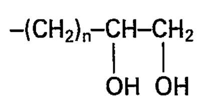
Va

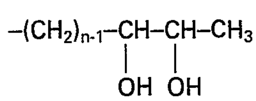
Vb

ou bien

d) qu'on le transforme directement avec des agents d'alkylation de formule (Xa) ou (Xb)

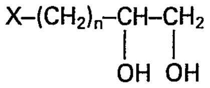

Xa

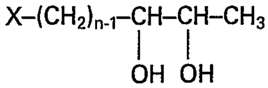

Xb

dans lesquelles n est égal à 2–6 et X a la signification donnée plus haut, en dihydroxy-akylxanthine contenant l'élément structural caractérisé par la formule (Va) ou (Vb), et que l'on transforme ensuite les mono-alkyl-dihydroxy-alkyl-xanthines obtenues suivant c) ou d), qui comportent encore un atome d'hydrogène en position $R^{1'''}$, $R^{2''}$ ou $R^{3''}$, éventuellement en présence de bases ou sous forme de leurs sels, avec des agents d'alkylation de formule

$R^4$–X (VI)

dans laquelle X et $R^4$ ont les significations définies pour la formule (VI), en composés de formule (I), ou bien que les xanthines dialkylées préparées suivant c), contenant l'élément structural de formule (IXa) ou (IXb), sont d'abord alkylées avec les composés de formule $R^4$–X (VI) et que l'on ouvre ensuite par hydrolyse de cycle dioxolanne avec formation des dihydroxy-alkylxanthines de formule (I).

8. Composés de formule (XI)

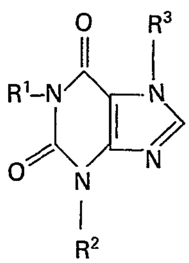

(XI)

dans laquelle l'un des radicaux $R^1$, $R^2$ et $R^3$ représente un groupe de formule (IVa) ou (IVb)

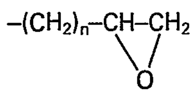

IVa

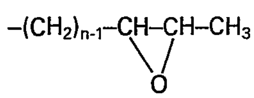

IVb

ou (IXa) ou (IXb)

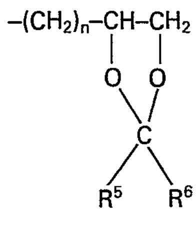

IXa

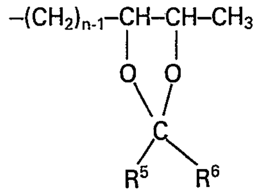

IXb

dans lesquelles n est égal à 2–6, $R^5$ et $R^6$ représentent chacun indépendamment l'un de l'autre un atome d'hydrogène, un groupe méthyle, phénylalkyle contenant jusqu'à 2 atomes de carbone dans le radical alkyle, ou phényle, les autres des radicaux $R^1$, $R^2$ et $R^3$ représentent indépendamment l'un de l'autre des atomes d'hydrogène ou des groupes alkyle ayant de 1 à 12 atomes de carbone dans les positions $R^1$ et $R^3$ et de 1 à 4 atomes de carbone dans la position $R^2$, la somme des atomes de carbone de ces deux radicaux étant au maximum égale à 14.

9. Composés selon la revendication 8, dans lesquels $R^1$ ou $R^3$ est le radical de formule (IVa) ou (IVb) et les autres radicaux sont, indépendamment l'un de l'autre, des groupes alkyle ayant de 1 à 4 atomes de carbone.

10. Composés selon la revendication 8, dans lesquels $R^1$ est un atome d'hydrogène, un groupe alkyle ayant de 1 à 5 atomes de carbone ou un radical de formule (IXa) ou (IXb) et $R^1$ et $R^3$ sont chacun, indépendamment l'un de l'autre, un groupe alkyle ayant de 1 à 3 atomes de carbone ou un radical de formule (IXa) ou (IXb).

**Revendications pour l'Etat contractant AT**

1. Procédé pour la préparation de dérivés de xanthine de formule (I)

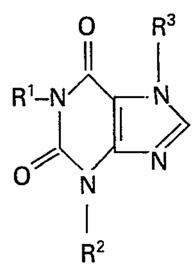

I

dans laquelle l'un des radicaux $R^1$, $R^2$ ou $R^3$ représente un groupe dihydroxy-alkyle de formule $CH_2OH$–CHOH–$(CH_2)_n$– ou $CH_3$–CHOH–CHOH–$(CH_2)_{n-1}$– où n = 2–6, et les deux autres radicaux représentent des groupes alkyle à chaîne droite ou ramifiée contenant jusqu'à 12 atomes de carbone à la position de $R^1$ et de $R^3$ et jusqu'à 4 atomes de carbone à la position de $R^2$, la somme des atomes de carbone de ces deux substituants alkyle étant toutefois égale à 14 au maximum, et $R^2$ et $R^3$ n'étant pas simultanément des groupes méthyle lorsque $R^1$ représente un radical 4,5- ou 5,6-dihydroxy-hexyle, caractérisé par le fait que l'on transforme un composé de formule (II)

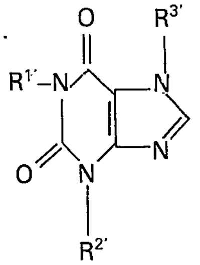

dans laquelle l'un des radicaux $R^{1'}$, $R^{2'}$ ou $R^{3'}$ est un groupe alcényle de formule (IIIa) ou (IIIb)

$-(CH_2)_n-CH=CH_2$ IIIa

$-(CH_2)_{n-1}-CH=CH-CH_3$ IIIb

dans lesquelles n est chaque fois égal à 2–6, et les deux autres substituants représentent chacun un atome d'hydrogène ou un groupe alkyle tel que défini pour la formule (I)

a) au niveau de la double liaison éthylénique, avec des oxydants appropriés, en une époxy-alkylxanthine contenant l'élément structural de formule (IVa) ou (IVb)

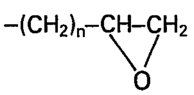 IVa

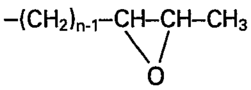 IVb

et que l'on ouvre par hydrolyse le cycle oxyrane de celui-ci avec formation d'une dihydroxy-alkylxanthine contenant l'élément structural caractéristique de formule (Va) ou (Vb)

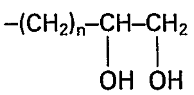 Va

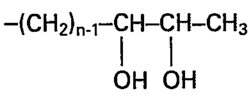 Vb

ou bien

b) qu'il est directement dihydroxylé avec des oxydants usuels au niveau de la double liaison éthylénique en dihydroxy-alkylxanthine contenant l'élément structural caractérisé par la formule (Va) ou (Vb) et qu'ensuite ceux des diols obtenus suivant a) ou b) qui comportent encore un atome d'hydrogène aux positions $R^{1'}$, $R^{2'}$ et/ou $R^{3'}$ sont alkylés en composés de formule (I), éventuellement en présence de bases ou sous forme de leurs sels, avec des agents d'alkylation de formule

$R^4-X$ (VI)

dans laquelle X représente un halogène ou un groupement ester sulfonique ou ester phosphorique et $R^4$ représente les radicaux alkyle définis pour la formule (I), ou bien que l'on transforme un composé de formule (VII)

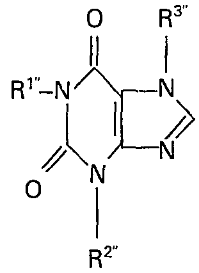

dans lequel deux au plus des substituants $R^{1''}$ à $R^{3''}$ représentent chacun un groupe alkyle défini pour la formule (I) et deux au plus de ces radicaux représentent des atomes d'hydrogène, éventuellement en présence de bases ou sous forme de ses sels,

c) avec des agents d'alkylation de formule (VIIIa) ou (VIIIb)

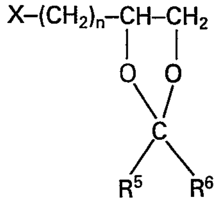 VIIIa

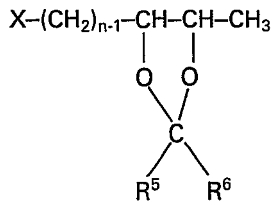 VIIIb

où n est chaque fois égal à 2–6, $R^5$ et $R^6$ représentant indépendamment l'un de l'autre un atome d'hydrogène, un groupe alkyle inférieur, phénylalkyle contenant jusqu'à 2 atomes de carbone dans le radical alkyle, ou un group phényle éventuellement substitué, et X représentant un halogène ou un groupement ester sulfonique ou ester phosphorique, en une xanthine di- ou tri-alkylée contenant l'élément structural de formule (IXa) ou (IXb)

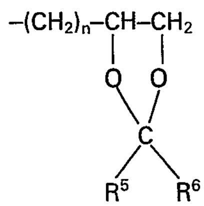 IXa

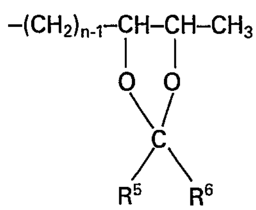 IXb

et que l'on ouvre par hydrolyse le cycle 1,3-dioxolanne de celui-ci avec séparation de $R^5-CO-R^6$ et formation d'une dihydroxy-alkylxanthine contenant l'élément structural caractéristique de formule (Va) ou (Vb)

$-(CH_2)_n-CH-CH_2$ Va
| |
OH OH

$-(CH_2)_{n-1}-CH-CH-CH_3$ Vb
| |
OH OH

ou bien

d) qu'on le transforme directement avec des agents d'alkylation de formule (Xa) ou (Xb)

$X-(CH_2)_n-CH-CH_2$ Xa
| |
OH OH

$X-(CH_2)_{n-1}-CH-CH-CH_3$ Xb
| |
OH OH

dans lesquelles n est égal à 2–6 et X a la signification donnée plus haut, en dihydroxy-aklylxanthine contenant l'élément structural caractérisé par la formule (Va) ou (Vb), et que l'on transforme ensuite les mono-akyl-dihydroxy-alkyl-xanthines obtenues suivant c) ou d), qui comportent encore un atome d'hydrogène en position $R^{1'''}$, $R^{2'''}$ ou $R^{3'''}$, éventuellement en présence de bases ou sous forme de leurs sels, avec des agents d'alkylation de formule

$R^4-X$ (VI)

dans laquelle X et $R^4$ on les significations définies pour la formule (VI), en composés de formule (I), ou bien que les xanthines dialkylées préparées suivant c), contenant l'élément structural de formule (IXa) ou (IXb), sont d'abord alkylées avec les composés de formule $R^4-X$ (VI) et que l'on ouvre ensuite par hydrolyse le cycle dioxolanne avec formation des dihyroxy-alkylxanthines de formule (I).

2. Procédé selon la revendication 1, caractérisé par le fait que l'on oxyde un composé de formule (II), dans lequel l'un des radicaux $R^1$, $R^2$ et $R^3$ est un groupe alcényle de formule (IIIa) ou (IIIb), avec un composé contenant un groupe peroxyde, en oxyrane, et que l'on hydrolyse celui-ci en milieu aqueux à une température de 20 à 100°C.

3. Procédé selon la revendication 2, caractérisé par le fait que l'on effectue l'oxydation avec des acides percarboxyliques à une température de −10 à 40°C.

4. Procédé selon la revendication 1, caractérisé par le fait que l'on oxyde un composé de formule (II) dans lequel l'un des radicaux $R^{1'}$, $R^{2'}$ et $R^{3'}$ est un groupe alcényle de formule (IIIa) ou (IIIb), avec du peroxyde d'hydrogène, du chlorure de chromyle, un permanganate, de l'iode, du bioxyde de sélénium, de l'oxyde VI de molybdène ou avec du tétroxyde d'osmium.

5. Procédé selon la revendication 4, caractérisé par le fait que l'oxydation est effectuée avec des quantités stoechiométriques de tétroxyde d'osmium et que l'on hydrolyse par voie de réduction l'ester complexe d'osmium (VI) intermédiaire formé.

6. Procédé selon la revendication 1, caractérisé par le fait que l'on ouvre par hydrolyse le dérivé de xanthine contenant le cycle dioxolanne, obtenue dans la technique c), en milieu aqueux, à des températures allant de 20°C au point d'ébullition du milieu réactionnel.

7. Procédé selon une ou plusierus des revendications 1 à 6, caractérisé par le fait que dans les composés de formule (I), $R^1$ ou $R^2$ représente un radical $CH_2OH-CHOH-(CH_2)_n-$ où n = 3 ou 4 et les deux substituants alkyle $R^2$ et $R^3$ ou $R^1$ et $R^3$ contiennent ensemble de 3 à 6 atomes de carbone.

8. Procédé selon une ou plusieurs des revendications 1 à 6, caractérisé par le fait que dans les composés de formule (I), $R^3$ représente un groupe $CH_2OH-CHOH-(CH_2)_n-$ où n = 2 à 5, ou un groupe 4,5-dihydroxy-hexyle, et la somme des atomes de carbone es deux radicaux alkyle $R^1$ et $R^2$ va de 3 à 7.

9. Procédé selon la revendication 8, caractérisé par le fait que les composés de formule (I) sont des 1,3-dialkyl-7-(5,6-dihydroxy-hexyl)-xanthines, mais préférablement la 3-éthyl-7-(5,6-dihydroxyhexyl)-1-propylxanthine.

10. Procédé pour la préparation d'un médicament, caractérisé par le fait que l'on prépare suivant le procédé d'une ou de plusieurs des revendications 1 à 9 un composé de formule (I), dans lequel l'un des radicaux $R^1$, $R^2$ ou $R^3$ représente un groupe dihydroxy-alkyle de formule $CH_2OH-CHOH-(CH_2)_n-$ ou $CH_3-CHOH-CHOH-(CH_2)_{n-1}-$ où n = 2–6 et les deux autres radicaux représentent des groupes alkyle à chaîne droite ou ramifiée ayant jusqu'à 12 atomes de carbone dans la position de $R^2$ et de $R^3$ et jusqu'à 4 atomes de carbone dans la position de $R^2$, la somme des atomes de carbone de ces deux substituants alkyle étant toutefois égale à 14 au maximum et que l'on confectionne celui-ci avec un adjuvant acceptable du point de vue thérapeutique.